(19) Europäisches Patentamt
European Patent Office
Office européen des brevets

(11) Veröffentlichungsnummer: **0 059 387**
**B1**

(12) # EUROPÄISCHE PATENTSCHRIFT

(45) Veröffentlichungstag der Patentschrift: **08.05.85**

(21) Anmeldenummer: **82101269.7**

(22) Anmeldetag: **19.02.82**

(51) Int. Cl.[4]: **C 07 D 487/14,** C 07 D 495/22, C 07 D 487/04, A 61 K 31/55 // (C07D487/14, 243:00, 235:00, 205:00),(C07D495/22, 333:00, 243:00, 235:00, 205:00),(C07D487/04, 243:00, 205:00)

(54) **Imidazodiazepine, Verfahren und Zwischenprodukte zu ihrer Herstellung, sowie diese enthaltende Arzneimittel.**

(30) Priorität: **27.02.81 CH 1339/81**

(43) Veröffentlichungstag der Anmeldung: **08.09.82 Patentblatt 82/36**

(45) Bekanntmachung des Hinweises auf die Patenterteilung: **08.05.85 Patentblatt 85/19**

(84) Benannte Vertragsstaaten: **BE CH DE FR GB IT LI NL SE**

(56) Entgegenhaltungen: **EP - A - 0 027 214**

(73) Patentinhaber: **F. HOFFMANN-LA ROCHE & CO. Aktiengesellschaft, CH-4002 Basel (CH)**

(72) Erfinder: **Hunkeler, Walter, Dr., Im Stigler 32, CH-4465 Magden (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 059 387 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Imidazodiazepine. Im speziellen betrifft sie Imidazodiazepine der allgemeinen Formel

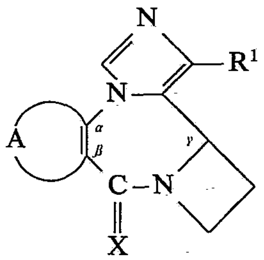

(I)

worin

A zusammen mit den beiden mit $\alpha$ und $\beta$ bezeichneten Kohlenstoffatomen die Gruppe

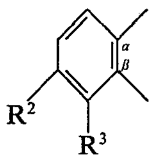 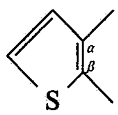

(a) oder (b)

$R^1$ Wasserstoff, niederes Alkyl, niederes Alkoxymethyl, Halogen, Nitro oder einen Rest der Formel $-COOR^4$,
$R^2$ Wasserstoff, Trifluormethyl oder Halogen,
$R^3$ Wasserstoff, Trifluormethyl, Halogen oder niederes Alkyl,
$R^4$ Methyl, Äthyl oder Isopropyl und
X ein Sauerstoff- oder Schwefelatom bedeuten und das mit
$\gamma$ bezeichnete Kohlenstoffatom die (S)- oder (R,S)-Konfiguration

aufweist,

und pharmazeutisch annehmbare Säureadditionssalze davon.

Diese Verbindungen sind neu; sie zeichnen sich durch wertvolle pharmakodynamische Eigenschaften aus und eignen sich demnach zur Bekämpfung bzw. Verhütung von Krankheiten.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine Verbindung der allgemeinen Formel I oder ein pharmazeutisch annehmbares Säureadditionssalz davon, und die Herstellung solcher Arzneimittel.

Der Ausdruck »niederes Alkyl« bezeichnet gesättigte Kohlenwasserstoffreste, welche geradkettig oder verzweigt sein können, mit höchstens 7, vorzugsweise höchstens 4 Kohlenstoffatomen, wie Methyl, Äthyl, Isopropyl, t-Butyl und dergleichen. Der Ausdruck »niederes Alkoxymethyl« umfaßt Reste, wie Methoxymethyl, Äthoxymethyl und dergleichen. Der Ausdruck »Halogen« bedeutet die vier Formen Fluor, Chlor, Brom und Jod.

$R^1$ bedeutet vorzugsweise Wasserstoff, Chlor oder einen Rest der Formel $-COOR^4$, wobei $R^4$ vorzugsweise Äthyl bedeutet. Das Symbol A bedeutet vorzugsweise die eingangs dargestellte Gruppe (a); dabei bedeuten $R^2$ vorzugsweise Wasserstoff und $R^3$ vorzugsweise Wasserstoff oder Chlor. Das Symbol X bedeutet vorzugsweise ein Sauerstoffatom.

Eine ganz besonders bevorzugte Verbindung im Rahmen der vorliegenden Erfindung ist Äthyl-(S)-8-chlor-12,12a-dihydro-9-oxo-9H,11H-azeto[2,1-c]imidazo[1,5-a][1,4]benzodiazepin-1-carboxylat.

Weitere im Rahmen der vorliegenden Erfindung besonders bevorzugte, von der allgemeinen Formel I umfaßte Verbindungen sind:

Äthyl-(R,S)-12,12a-dihydro-9-oxo-9H,11H-azeto[2,1-c]imidazo[1,5-a][1,4]benzodiazepin-1-carboxylat,
(S)-8-Chlor-12,12a-dihydro-9H,11H-azeto[2,1-c]imidazo[1,5-a][1,4]benzodiazepin-9-on,
Äthyl-(S)-12,12a-dihydro-9-oxo-9H,11H-azeto[2,1-c]imidazo[1,5-a][1,4]benzodiazepin-1-carboxylat und
(S)-1-Chlor-12,12a-dihydro-9H,11H-azeto[2,1-c]imidazo[1,5-a][1,4]benzodiazepin-9-on.

Die Imidazodiazepine der allgemeinen Formel I und ihre pharmazeutisch annehmbaren Säureadditionssalze können erfindungsgemäß hergestellt werden, indem man

a) eine Verbindung der allgemeinen Formel

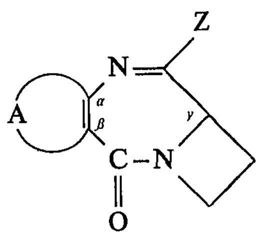

(II)

worin

A obige Bedeutung besitzt und
Z eine Abgangsgruppe bedeutet,

in Gegenwart einer Base mit einem Isocyanessigester der allgemeinen Formel

$CN—CH_2—COOR^4$ (III)

worin $R^4$ obige Bedeutung besitzt,

umsetzt, oder

b) eine Verbindung der allgemeinen Formel

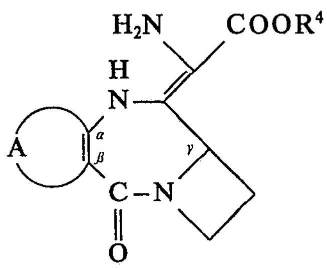

(IV)

worin A und $R^4$ obige Bedeutung besitzen,

mit einem Formylierungsmittel behandelt, oder

c) eine Verbindung der allgemeinen Formel

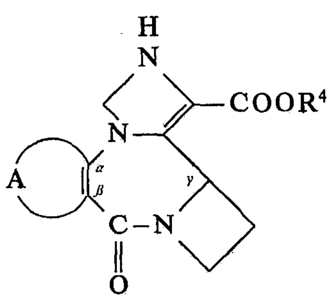

(V)

oder

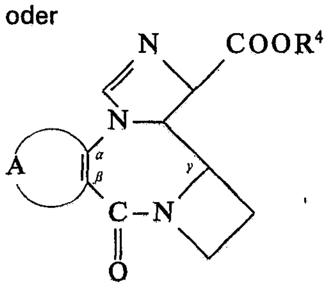

(VI)

worin A und $R^4$ obige Bedeutung besitzen,

dehydriert, oder

d) in einer Aminoverbindung der allgemeinen Formel

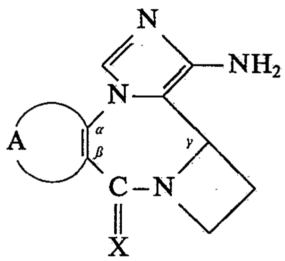

(VII)

worin A und X obige Bedeutung besitzen,

die Aminogruppe durch ein Halogenatom oder durch die Nitrogruppe ersetzt, oder

e) in einer Aminoverbindung der obigen allgemeinen Formel VII die Aminogruppe zur Nitrogruppe oxidiert, oder

f) eine Carbonsäure der allgemeinen Formel

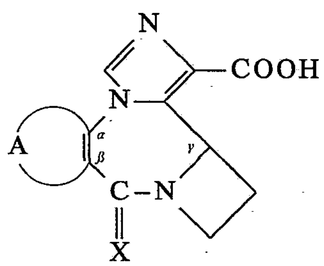

(VIII)

worin A und X obige Bedeutung besitzen,

decarboxyliert, oder

g) eine Verbindung der allgemeinen Formel

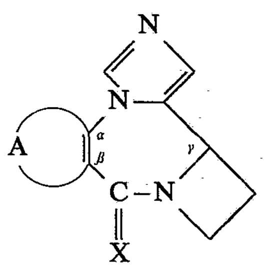

(Ia)

worin A und X obige Bedeutung besitzen,

am Imidazolring halogeniert, oder

h) in einer Verbindung der allgemeinen Formel

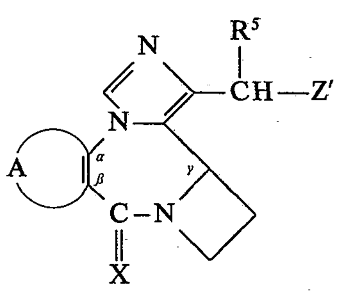

(IX)

worin

A und X obige Bedeutung besitzen,
$R^5$ Wasserstoff oder niederes Alkyl und
Z' eine Abgangsgruppe bedeuten,

die mit Z' bezeichnete Abgangsgruppe unter reduktiven Bedingungen abspaltet, oder

i) eine Verbindung der allgemeinen Formel

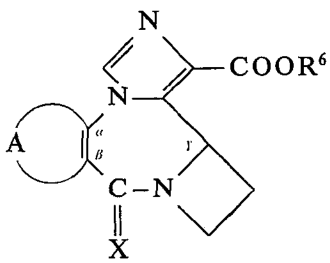

(Ib)

worin

A und X obige Bedeutung besitzen und
$R^6$ niederes Alkyl bedeutet,

umestert, oder

j) in einer Verbindung der allgemeinen Formel

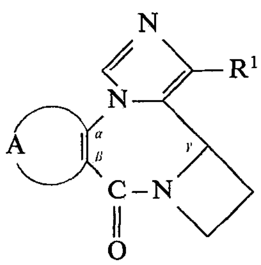

(Ic)

worin $R^1$ und A obige Bedeutung besitzen,

die Carbonylgruppe in die Thiocarbonylgruppe überführt, oder

k) eine Verbindung der allgemeinen Formel

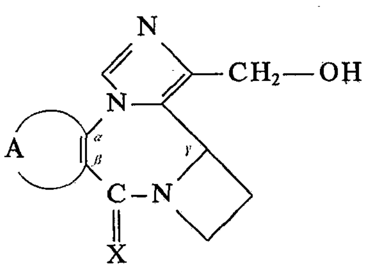

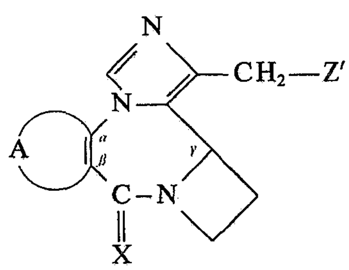

(XVI) bzw. (IXa)

worin A, X und Z' obige Bedeutung besitzen,

mit einem einen niederen Alkylrest liefernden Alkylierungsmittel, bzw. mit einem niederen Alkohol veräthert, und

l) erwünschtenfalls eine erhaltene Verbindung der allgemeinen Formel I in ein pharmazeutisch annehmbares Säureadditionssalz überführt.

Gemäß Verfahrensvariante a) können Verbindungen der allgemeinen Formel I demnach aus Verbindung der allgemeinen Formel II und Isocyanessigestern der allgemeinen Formel III hergestellt werden. Die in Formel II mit Z bezeichnete Abgangsgruppe ist beispielsweise eine leicht abspaltbare Phosphinylgruppe, z. B. eine Gruppe der Formel

$$-O\overset{\overset{O}{\|}}{P}(OR^7)_2 \quad \text{oder} \quad -O\overset{\overset{O}{\|}}{P}(NR^8R^9)_2$$

worin

$R^7$ niederes Alkyl und
$R^8$ und $R^9$ je niederes Alkyl, Allyl, Phenyl oder substituiertes Phenyl oder zusammen mit dem Stickstoffatom einen unsubstituierten oder substituierten heterocyclischen Ring mit 3—8 Gliedern (wie Morpholin)

bedeuten,

ein Halogenatom, eine Alkylthiogruppe, eine Aralkylthiogruppe, eine N-Nitrosoalkylaminogruppe, eine Alkoxygruppe, eine Mercaptogruppe und dergleichen (wenn Z eine Mercaptogruppe bedeutet, so handelt es sich bei der entsprechenden Verbindung der Formel II um die Iminothiol-Form des entsprechenden Thiolactams). Die Umsetzung der Verbindungen der Formeln II und III erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um das Anion des Isocyanessigesters der Formel III zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmäßigerweise zwischen etwa −40°C und etwa Raumtemperatur.

Gemäß Verfahrensvariante b) können Verbindungen der allgemeinen Formel I dadurch hergestellt werden, daß man Verbindungen der allgemeinen Formel IV mit einem Formylierungsmittel behandelt. Als Formylierungsmittel eignen sich für diesen Verfahrensaspekt niedere Alkylester der ortho-Ameisensäure und technische Äquivalente davon, beispielsweise ortho-Amide, wie N,N-Dimethylformamid-dimethylacetal, N,N,N′,N′,N″,N″-Hexamethylmethantriamin und dergleichen. Die Reaktion von Verbindungen der allgemeinen Formel IV mit dem Formylierungsmittel erfolgt zweckmäßigerweise in Gegenwart eines sauren Katalysators, beispielsweise einer organischen oder anorganischen Säure, wie p-Toluolsulfonsäure, Phosphorsäure und dergleichen, und bei Raumtemperatur oder oberhalb davon, beispielsweise zwischen etwa 25° und etwa 150°C.

Gemäß Verfahrensvariante c) können Verbindungen der allgemeinen Formel I durch Dehydrierung von Verbindungen der allgemeinen Formel V oder VI hergestellt werden. Bevorzugte Reagenzien für diese Dehydrierung umfassen Mangandioxid, Palladium auf Kohle und elementaren Sauerstoff, wobei Luft-Sauerstoff bereits genügt, doch kann auch beispielsweise Kaliumpermanganat verwendet werden. Lösungsmittel, welche für diese Dehydrierung verwendet werden können, umfassen chlorierte Kohlenwasserstoffe, wie Methylenchlorid und Chloroform, aromatische Kohlenwasserstoffe, Dimethylformamid usw. Die Dehydrierung erfolgt bei Raumtemperatur oder oberhalb davon, zweckmäßigerweise zwischen etwa 25° und etwa 200°C.

Gemäß Verfahrensvariante d) können Verbindungen der allgemeinen Formel I, worin $R^1$ Halogen oder Nitro bedeutet, hergestellt werden, indem man in einer Verbindung der allgemeinen Formel VII die Aminogruppe durch ein Halogenatom oder die Nitrogruppe ersetzt. Zweckmäßigerweise geht man dabei so vor, daß man die Aminoverbindung der Formel VII in ein entsprechendes Diazoniumsalz überführt und dieses, gegebenenfalls ohne vorgängige Isolierung, mit einem Nitrit, wie Natriumnitrit, oder mit einem Halogenid, z. B. mit einem Chlorid oder Bromid, in Gegenwart eines Kupfer(I)salzes umsetzt. Für die Herstellung der entsprechenden Jodide ist die Anwesenheit eines Kupfer(I)salzes nicht erforderlich. Entsprechende Fluoride werden zweckmäßigerweise über die entsprechenden Diazonium-tetrafluoroborate hergestellt, beispielsweise durch Bestrahlen mit UV-Licht. Diese Reaktionen werden vorzugsweise in wäßrigen Lösungen bei Temperaturen von etwa −10°C bis etwa Raumtemperatur durchgeführt.

Gemäß Verfahrensvarinate e) können Verbindungen der allgemeinen Formel I, worin $R^1$ Nitro bedeutet, auch hergestellt werden, indem man eine Aminoverbindung der Formel VII oxidiert. Als Oxidationsmittel eignen sich beispielsweise Persäuren, wie Peressigsäure, Trifluorperessigsäure, m-Chlorperbenzoesäure und Perbenzoesäure, und dergleichen. Als Lösungsmittel kommen, je nach eingesetztem Oxidationsmittel, Carbonsäuren, wie Essigsäure etc., halogenierte Kohlenwasserstoffe, wie Methylenchlorid, Chloroform, 1,2-Dichloräthan etc., oder dergleichen in Frage. In der Regel arbeitet man bei Temperaturen von etwa 0°C bis etwa Raumtemperatur.

Gemäß Verfahrensvariante f) können Verbindungen der allgemeinen Formel I, worin $R^1$ Wasserstoff

bedeutet, durch Decarboxylierung von Carbonsäuren der allgemeinen Formel VIII hergestellt werden. Diese Reaktion erfolgt zweckmäßigerweise durch trockenes Erhitzen der gegebenenfalls rohen Carbonsäure der allgemeinen Formel VIII auf Temperaturen von etwa 150°C bis etwa 400°C, wobei die Reaktionstemperatur vom Schmelzpunkt der jeweils eingesetzten Verbindung der Formel VIII abhängt.

Gemäß Verfahrensvariante g) können Verbindungen der allgemeinen Formel I, worin $R^1$ Halogen bedeutet, durch Halogenierung von Verbindungen der allgemeinen Formel Ia hergestellt werden. Geeignete Halogenierungsmittel sind beispielsweise N-Chlorsuccinimid, N-Bromsuccinimid, N-Chloracetamid, N-Bromacetamid und elementares Jod. Als Lösungsmittel verwendet man zweckmäßigerweise inerte organische Lösungsmittel, beispielsweise halogenierte Kohlenwasserstoffe, wie Methylenchlorid, 1,2-Dichloräthan, Chloroform und dergleichen, Dimethylformamid, Dimethylacetamid, Acetonitril, Äther, wie Diäthyläther, Tetrahydrofuran, Dioxan und dergleichen, usw. Die Halogenierung kann je nach eingesetztem Lösungsmittel in einem Temperaturbereich von etwa 0°C bis etwa 120°C durchgeführt werden.

Gemäß Verfahrensvariante h) können Verbindungen der allgemeinen Formel I, worin $R^1$ niederes Alkyl bedeutet, hergestellt werden, indem man in einer Verbindung der allgemeinen Formel IX die mit Z' bezeichnete Abgangsgruppe unter reduktiven Bedingungen abspaltet. Diese Reaktion erfolgt nach an sich bekannten Methoden, wobei die Wahl der geeigneten Abgangsgruppe Z' sowie die Ermittlung der für die Abspaltung geeigneten Reaktionsbedingungen, unter welchen andere im Molekül enthaltene Strukturelemente nicht angegriffen werden dürfen, dem Fachmann keinerlei Schwierigkeiten bieten. Für den vorliegenden Verfahrensaspekt besonders geeignete Abgangsgruppen sind beispielsweise Halogenatome, wie Chlor, Brom und Jod, welche sich unter hydrogenolytischen Bedingungen leicht abspalten lassen, beispielsweise durch Behandeln mit elementarem Wasserstoff in Gegenwart eines geeigneten Katalysators (z.B. Palladium/Kohle, Raney-Nickel, etc.) in einem inerten organischen Lösungsmittel. Geeignete Lösungsmittel sind beispielsweise Alkohole, wie Methanol, Äthanol und Isopropanol, Äther, wie Diäthyläther, Tetrahydrofuran, Dioxan und Dimethoxyäthan, und dergleichen. Je nach Reaktivität des eingesetzten Katalysators arbeitet man bei Drucken von etwa Normaldruck bis etwa 300 bar und bei Temperaturen von etwa Raumtemperatur bis etwa 150°C.

Gemäß Verfahrensvariante i) können Verbindungen der allgemeinen Formel I durch Umesterung von Verbindungen der allgemeinen Formel Ib hergestellt werden, d. h. daß in Verbindungen der allgemeinen Formel Ib der mit $R^6$ bezeichnete Alkylrest gegen den gewünschten Rest $R^4$ ausgetauscht wird.

Diese Umesterung erfolgt in an sich bekannter Weise durch Umsetzen einer Verbindung der allgemeinen Formel Ib mit einem dem gewünschten Rest $R^4$ entsprechenden Alkohol, d. h. mit Methanol, Äthanol oder Isopropanol, bei Raumtemperatur oder unter Erwärmen auf eine Temperatur von etwa 25° bis 150°C. Vorzugsweise wird die Umesterung in Gegenwart einer Base durchgeführt, wobei sich im vorliegenden Fall insbesondere Kaliumcyanid oder ähnliche schwache Basen eignen. Als Base eignet sich aber auch das dem gewünschten Rest $R^4$ entsprechende Alkoholat, d. h. z. B. Natriummethanolat, -äthanolat oder -isopropanolat, oder die entsprechenden Kaliumsalze. Als Lösungsmittel dient vorzugsweise der dem Rest $R^4$ im gewünschten Endprodukt der Formel I entsprechende Alkohol; die Umesterung kann jedoch auch in einem inerten organischen Lösungsmittel erfolgen, beispielsweise in einem aromatischen Kohlenwasserstoff, wie Benzol oder Xylol, einem Äther, wie Dioxan, Tetrahydrofuran oder Äthylenglykol-dimethyläther, in Dimethylformamid, in Dimethylsulfoxid oder dergleichen. Bei dieser Umesterung kann man nicht nur einen niedriger siedenden Alkohol durch einen höher siedenden Alkohol, sondern auch einen höher siedenden Alkohol durch einen niedriger siedenden Alkohol ersetzen.

Die Umesterung kann indessen ohne weiteres auch mehrstufig durchgeführt werden, beispielsweise dadurch, daß man die eingesetzte Verbindung der Formel Ib zur entsprechenden freien Carbonsäure der Formel VIII hydrolysiert, aus dieser ein reaktionsfähiges funktionelles Derivat (z.B. ein Säurechlorid oder dergleichen) herstellt und anschließend dieses reaktionsfähige Carbonsäurederivat mit dem der Bedeutung von $R^4$ in der gewünschten Verbindung der Formel I entsprechenden Alkohol zur Reaktion bringt.

Gemäß Verfahrensvariante j) können Verbindungen der allgemeinen Formel Ic in entsprechende Verbindungen der Formel I, worin X ein Schwefelatom bedeutet, übergeführt werden, und zwar durch Behandeln mit einem Schwefelungsmittel, was in an sich bekannter Weise erfolgen kann. Beispielsweise kann man als Schwefelungsmittel Phosphorpentasulfid verwenden, wobei dieses vorzugsweise im Überschuß eingesetzt wird und die Reaktion vorteilhafterweise in einem inerten organischen Lösungsmittel, wie Dioxan, Methylenchlorid oder dergleichen, in Gegenwart von Triäthylamin bei einer Temperatur von etwa 50°C bis zur Rückflußtemperatur des Reaktionsgemisches erfolgt. Als Schwefelungsmittel eignen sich indessen auch Verbindungen wie 2,4-Bis-(p-methoxyphenyl)-1,3,2,4-dithiaphosphetan-2,4-disulfid; derartige Schwefelungsmittel werden ungefähr in der berechneten Menge eingesetzt, und die Reaktion erfolgt in Gegenwart eines inerten Lösungsmittels, wie Toluol, Xylol, zweckmäßigerweise bei Rückflußtemperatur des Reaktionsgemisches oder in Hexamethyl-phosphorsäuretriamid zwischen etwa 60 und 110°C.

Gemäß Verfahrensvariante k) können Verbindungen der allgemeinen Formel I, worin $R^1$ niederes Alkoxymethyl bedeutet, hergestellt werden, indem man einen Alkohol der allgemeinen Formel XVI mit einem einen niederen Alkylrest liefernden Alkylierungsmittel oder eine Verbindung der allgemeinen

Formel IXa mit einem niederen Alkohol veräthert. Diese Reaktion erfolgt in einem inerten organischen Lösungsmittel, wie Dimethylformamid, Hexamethylphosphorsäuretriamid, Dimethylsulfoxid, Tetrahydrofuran oder irgend einem anderen geeigneten organischen Lösungsmittel, und in Gegenwart einer Base, die genügend stark basisch ist, um aus dem Alkohol der Formel XVI, bzw. aus dem niederen Alkohol das entsprechende Alkoholat zu bilden. Als Basen eignen sich z. B. Alkalimetallhydride, wie Natriumhydrid, Alkalimetalle, wie Natrium, und Alkalimetallamide, wie Lithiumamid und Lithiumdiisopropylamid. Geeignete Alkylierungsmittel sind z. B. Alkylhalogenide, wie Methyljodid, Äthyljodid und Äthylbromid, und Dialkylsulfate, wie Dimethylsulfat und Diäthylsulfat. Die Reaktionstemperatur liegt zweckmäßigerweise zwischen etwa 0°C und etwa 50°C.

Gemäß Verfahrensvariante l) können Verbindungen der allgemeinen Formel I erfindungsgemäß in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der allgemeinen Formel II können ausgehend von Verbindungen der Formel

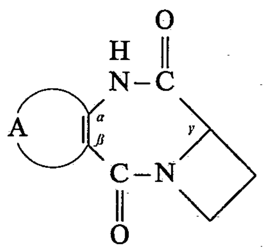

(X)

worin A obige Bedeutung besitzt,

hergestellt werden, und zwar nach Methoden, welche an sich bekannt sind, vgl. z. B. die Belgischen Patentschriften No. 802 233, 833 249 und 865 653, die Amerikanische Patentschrift No. 3 681 341 und J. Org. Chemistry 29, 231 (1964).

Verschiedene der weiter unten folgenden Beispiele enthalten detaillierte Angaben betreffend die Herstellung von Verbindungen der allgemeinen Formel II aus Verbindungen der allgemeinen Formel X.

Die Verbindungen der Formel X ihrerseits können nach an sich bekannten Methoden leicht hergestellt werden, beispielsweise durch Umsetzen eines entsprechenden Carbonsäureanhydrids der allgemeinen Formel

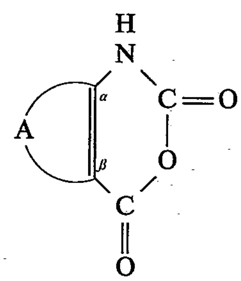

(XI)

worin A obige Bedeutung besitzt,

mit (L)- oder (D,L)-Azetidincarbonsäure,

Es ist auch möglich eine Verbindung der Formel

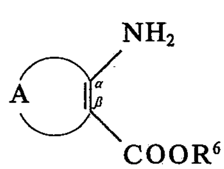

(XII)

worin A und $R^6$ obige Bedeutung besitzen,

mit einem reaktiven Derivat einer Carbonsäure der allgemeinen Formel

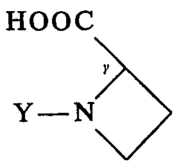

(XIII)

worin Y eine Schutzgruppe bedeutet,

beispielsweise einem Carbonsäurechlorid oder dergleichen, umzusetzen. Nach Entfernen der mit Y bezeichneten Schutzgruppe aus einer so erhaltenen Verbindung der allgemeinen Formel

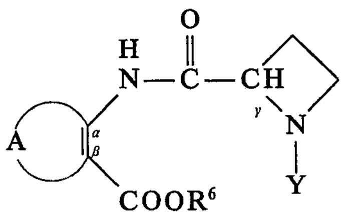

(XIV)

worin $R^6$, A und Y obige Bedeutung besitzen,

und Cyclisieren des erhaltenen Stoffes (beispielsweise durch kurzzeitiges Erhitzen auf Temperaturen von etwa 100° bis etwa 300°C) erhält man eine Verbindung der allgemeinen Formel X.

Die Verbindungen der allgemeinen Formel IV, V und VI lassen sich nach an sich bekannten Methoden (vgl. die Belgischen Patentschriften Nos. 833 248 und 839 364) ausgehend von Verbindungen der allgemeinen Formel II gemäß nachfolgendem Formelschema 1, worin A, $R^4$ und Z obige Bedeutung besitzen, herstellen:

Formelschema 1

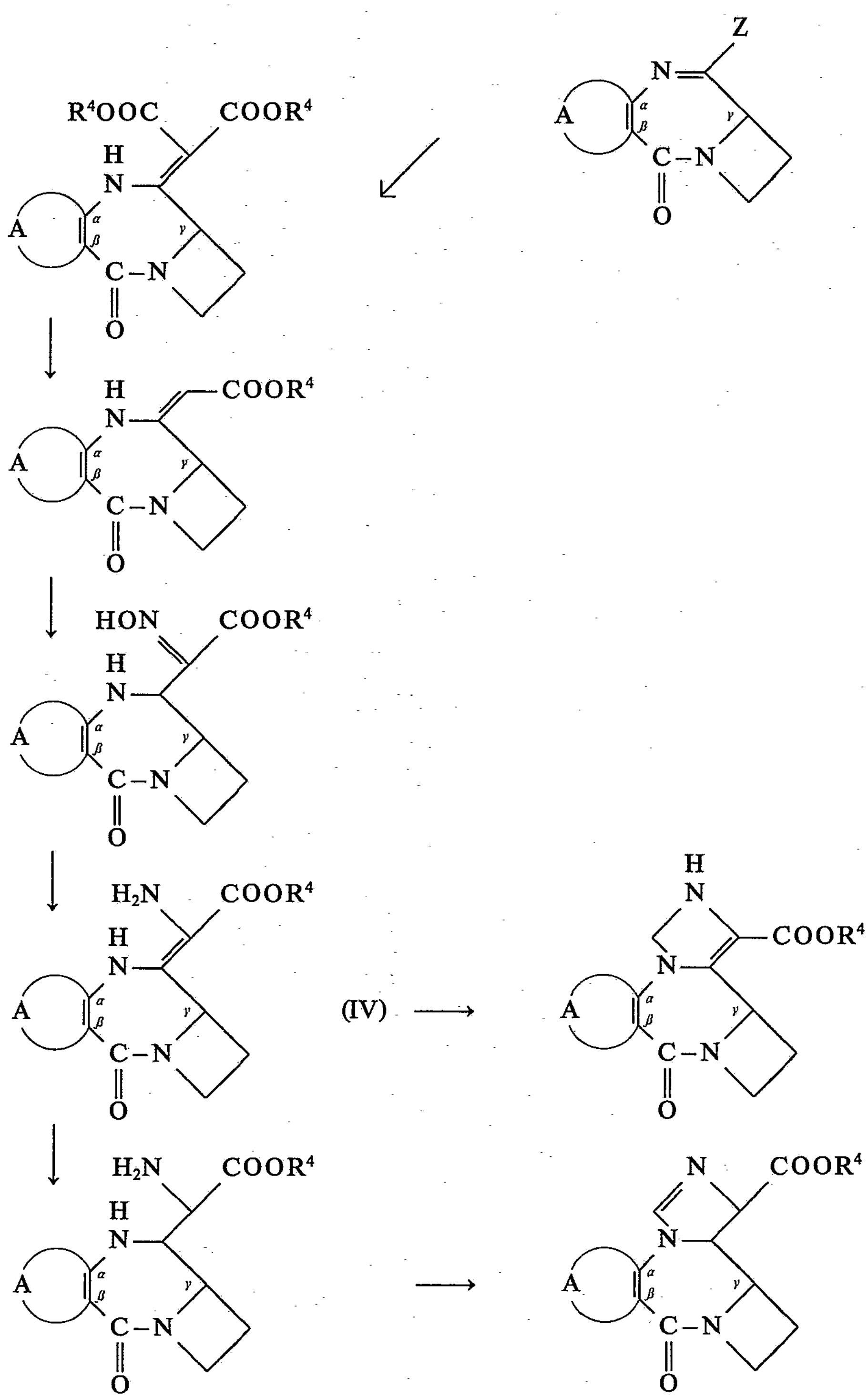

(II)

(IV)

(V)

(VI)

Die als Ausgangsstoffe verwendeten Aminoverbindungen der Formel VII können beispielsweise in an sich bekannter Weise aus den Carbonsäureaziden der Formel

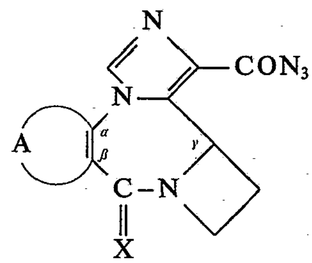

(XV)

worin A und X obige Bedeutung besitzen,

hergestellt werden, z. B. durch Behandeln einer solchen Verbindung mit einem Alkohol, wie Methanol, Äthanol, Benzylalkohol oder dergleichen, bei erhöhter Temperatur und Hydrolyse des erhaltenen Urethans. In einer bevorzugten Ausführungsform verwendet man Benzylalkohol und spaltet das erhaltene Benzylurethan hydrogenolytisch.

Die Carbonsäureazide der Formel XV können beispielsweise durch Behandeln eines Carbonsäureesters der Formel Ib mit Hydrazin und Umsetzen des erhaltenen Carbonsäurehydrazides mit Natriumnitrit in Gegenwart einer Säure, wie Essigsäure, hergestellt werden.

Die als Ausgangsstoffe verwendeten Verbindungen der allgemeinen Formel VIII können durch Hydrolyse von Carbonsäureestern der allgemeinen Formel Ib leicht hergestellt werden. Zweckmäßigerweise erfolgt die Hydrolyse in basischer, wäßriger Lösung, z. B. in wäßriger Natronlauge, gegebenenfalls in Gegenwart eines Lösungsmittels (wie z. B. Methanol, Äthanol, Tetrahydrofuran, Dioxan oder dergleichen). Handelt es sich bei der Verbindung der Formel Ib um einen t-Alkylester, z. B. um einen t-Butylester, so wird die Hydrolyse zweckmäßigerweise unter sauren Bedingungen durchgeführt; beispielsweise unter Verwendung von Trifluoressigsäure, oder wäßrigen Mineralsäuren, oder dergleichen.

Die als Ausgangsstoffe verwendeten Verbindungen der Formel IX sind aus Carbonsäureestern der Formel Ib, gemäß nachfolgendem Formelschema 2, worin A, X, Z' und $R^6$ obige Bedeutung besitzen und $R^{51}$ niederes Alkyl bedeutet, leicht zugänglich:

Formelschema 2

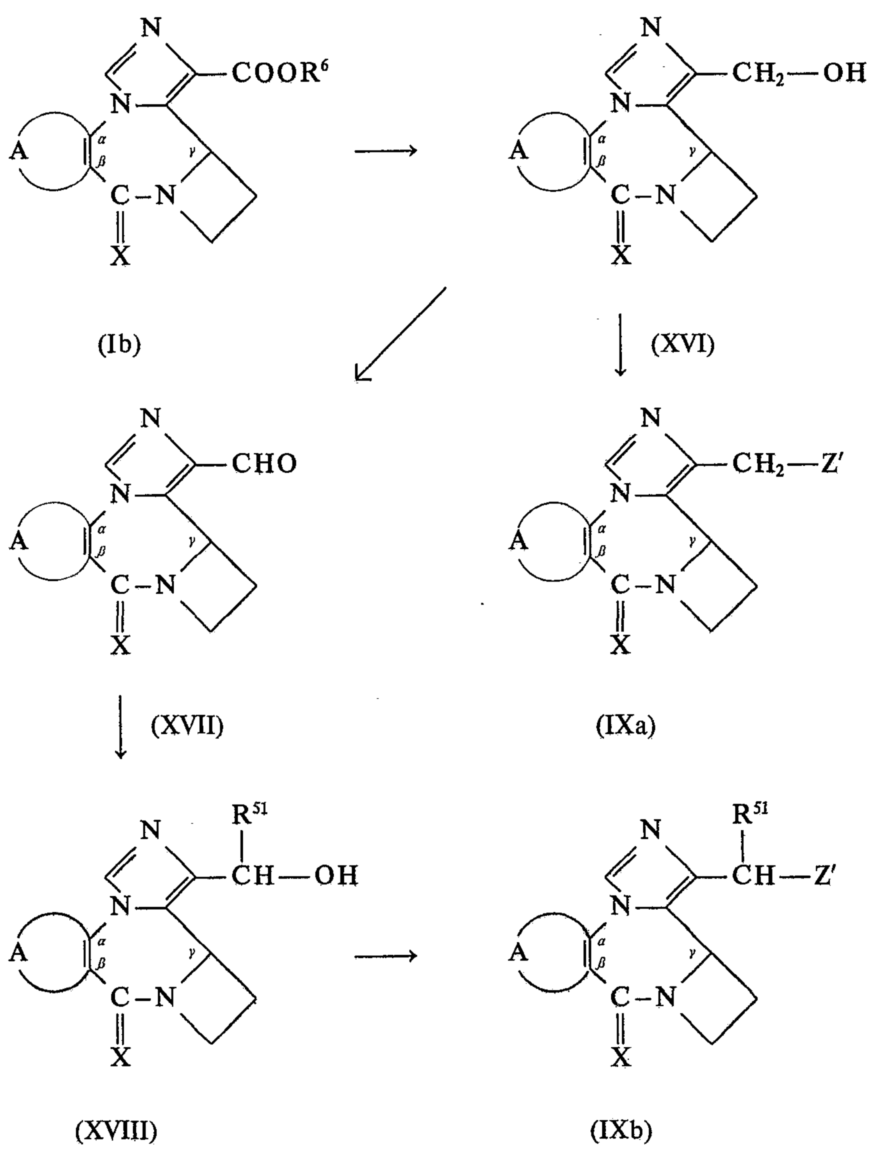

(Ib) (XVI) (XVII) (IXa) (XVIII) (IXb)

Die Reduktion einer Verbindung der Formel Ib zu einem Alkohol der Formel XVI erfolgt vorzugsweise mit einem Reduktionsmittel, wie Lithiumborhydrid, in einem inerten organischen Lösungsmittel, wie Diäthyläther, Tetrahydrofuran, Dimethoxyäthan oder dergleichen.

Die Herstellung einer Verbindung der Formel XVII aus einem Alkohol der Formel XVI erfolgt vorzugsweise mit einem milden Oxidationsmittel, wie Mangandioxid oder dergleichen, in einem inerten organischen Lösungsmittel, wie Methylenchlorid, Chloroform oder dergleichen.

Die Verbindungen der Formel XVIII können hergestellt werden, indem man eine Verbindung der allgemeinen Formel XVII nach allgemein bekannten und jedem Fachmann geläufigen Methoden mit einer den Rest $R^{51}$ liefernden metallorganischen Verbindung umsetzt. Als metallorganische Verbindung kommen in erster Linie Grignard-Verbindungen, wie Methyl-magnesiumjodid, Äthyl-magnesiumjodid, Isopropyl-magnesiumbromid, n-Propyl-magnesiumbromid, n-Butyl-magnesiumchlorid und dergleichen, in Frage. Als Lösungsmittel eignen sich Äther, wie Diäthyläther, Tetrahydrofuran, t-Butyl-methyläther, Mischungen davon, und dergleichen. Zweckmäßigerweise arbeitet man bei der Siedetemperatur des Reaktionsgemisches, man kann aber auch unterhalb davon arbeiten, beispielsweise bei Raumtemperatur.

Die Verbindungen der allgemeinen Formel IX, d. h. IXa und IXb, können nach allgemein bekannten und jedem Fachmann geläufigen Methoden aus Verbindungen der Formel XVI bzw. XVIII hergestellt werden. Entsprechende Halogenide erhält man beispielsweise durch Behandeln von Verbindungen der Formel XVI bzw. XVIII mit Halogenierungsmitteln, wie Thionylchlorid, Phosphoroxychlorid, Phosphorpentachlorid, Tetrabromkohlenstoff/Triphenylphosphin und dergleichen.

Auch die als Ausgangsstoffe verwendeten Verbindungen der allgemeinen Formeln II, IV, V, VI, VII, VIII, IX und XVI sind neu und ebenfalls Gegenstand der vorliegenden Erfindung.

Die Verbindungen der Formel Ib, worin $R^6$ nicht Methyl, Äthyl, Isopropyl oder t-Butyl bedeutet, sind ebenfalls Gegenstand der vorliegenden Erfindung.

Wie eingangs erwähnt, sind die Verbindungen der allgemeinen Formel I neu und besitzen äußerst wertvolle pharmakodynamische Eigenschaften. Sie weisen nur eine geringe Toxizität auf, und es hat sich gezeigt, daß sie eine ausgeprägte Affinität zu den zentralen Benzodiazepin-Rezeptoren haben und die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Affinität von Verbindungen der allgemeinen Formel I zu den zentralen Benzodiazepin-Rezeptoren wurde nach der in Life Science 20, 2101–2110 (1977) und Science 198, 849–851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ (»50% inhibiting concentration«) diejenige Konzentration der jeweiligen Testsubstanz, welche eine 50proz. Hemmung der spezifischen Bindung des tritiierten Diazepams an die spezifischen Benzodiazepin-Rezeptoren im cerebralen Cortex bewirkt.

Eine der typischen Eigenschaften von tranquilisierend wirksamen 1,4-Benzodiazepinen in Versuchstieren ist ihre ausgeprägte anticonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von tranquilisierend wirksamen 1,4-Benzodiazepinen zu antagonisieren vermögen.

In diesem Test verabreicht man Mäusen eine Stunde vor dem Pentetrazol (120 mg/kg, i.p.) 5 mg/kg (i.p.) Diazepam (d.h. eine supramaximale Dosis, die im Pentetrazol-Test an mehr als 900 Mäusen alle Versuchstiere vor krampfartigen Anfällen schützte) und 15 Minuten vor dem Pentetrazol die zu testende Verbindung p.o. Die antagonistische Wirksamkeit der untersuchten Verbindungen, d.h. ihr Vermögen, die Wirkung des Diazepams im Pentetrazol-Test aufzuheben, wird ermittelt, indem man die Mäuse auszählt, die in diesem Test krampfartige Anfälle erleiden.

In der nachstehenden Tabelle werden die Resultate dargestellt, welche mit repräsentativen Vertretern der durch die allgemeinen Formel I definierten Verbindungsklasse in dem vorstehend geschilderten Versuch erhalten worden sind. Angegeben wird für jede der darin figurierenden Verbindung der $ED_{50}$-Wert. Als $ED_{50}$ bezeichnet man die Menge der jeweiligen Testverbindung in mg/kg (p.o.), die bei 50% der Tiere den Diazepam-Effekt in obigem Versuch aufhebt. Außerdem enthält die Tabelle die oben definierten $IC_{50}$-Werte für alle darin angegebenen Testverbindungen.

Tabelle

| Verbindung der Formel I, worin A | $R^2$ | $R^3$ | $R^1$ | X | Konfiguration | $IC_{50}$ in nM/l | $ED_{50}$ in mg/kg p.o. |
|---|---|---|---|---|---|---|---|
| (a) | H | H | $-COOCH_2CH_3$ | O | (R,S) | 3,0 | 5,4 |
| (a) | H | H | $-COOCH_2CH_3$ | O | (S) | 1,3 | 3,7 |
| (a) | H | H | Cl | O | (S) | 37,0 | 6,9 |
| (a) | H | Cl | $-COOCH_2CH_3$ | O | (S) | 0,99 | 0,75 |
| (a) | H | Cl | H | O | (S) | 150 | 15,8 |

Wie bereits erwähnt, antagonisieren die Verbindungen der Formel I die zentral dämpfenden, muskelrelaxierenden, ataktischen, blutdrucksenkenden und atemdepressiven Eigenschaften von tranquilisierend wirksamen 1,4-Benzodiazepinen. Letztere stehen in der Therapie in vielfältigem Gebrauch und werden oft in hohen Dosierungen angewandt, sodaß die oben erwähnten Wirkungen auch als Nebenwirkungen stark hervortreten können. Die Verbindungen der Formel I können bei Intoxikationen, bei welchen übermäßige Einnahme von tranquilisierend wirksamen 1,4-Benzodiazepinen beteiligt ist, als Antidot verwendet werden. Sie eignen sich auch zur Abkürzung einer durch tranquilisierend wirksame 1,4-Benzodiazepine eingeleiteten Anästhesie in der Chirurgie und in der Geburtshilfe. Bei Neugeborenen kann eine eventuelle Atemdepression, die auf die Gabe von tranquilisierend wirksamen 1,4-Benzodiazepinen an die Mutter zurückgeht, aufgehoben werden. Die Verbindungen der Formel I können auch dazu verwendet werden, bei 1,4-Benzodiazepinen, die in anderen Indikationsgebieten eingesetzt werden, die in einem solchen Fall unerwünschten Wirkungen auf das zentrale Nervensystem zu unterdrücken. Beispiele derartiger, in anderen Indikationsgebieten einsetzbarer 1,4-Benzodiazepine sind die in den Britischen Patentschriften No. 1 444 529 und 1 474 305 beschriebenen schistosomizid wirksamen 1,4-Benzodiazepine, wie das (+)-5-(o-Chlorphenyl)-1,3-dihydro-3-methyl-7-nitro-2H-1,4-benzodiazepin-2-on.

Die Verbindungen der allgemeinen Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z. B. Lactose, Maisstärke oder Derivate davon, Talk, Stearinsäure oder deren Salze etc. verwenden. Für Weichgelatinekapseln eignen sich als Träger z. B. pflanzliche Öle, Wachse, Fette, halbfeste und flüssige Polyole etc. Zur Herstellung von Lösungen und Sirupen eignen sich als Träger z. B. Wasser, Polyole, Saccharose, Invertzucker, Glukose und dergleichen. Für Injektionslösungen eignen sich als Träger z. B. Wasser, Alkohole, Polyole, Glyzerin, pflanzliche Öle etc. Für Suppositorien eignen sich als Träger z. B. natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt, kann man Verbindungen der allgemeinen Formel I und pharmazeutisch annehmbare Säureadditionssalze davon erfindungsgemäß bei der Bekämpfung bzw. Verhütung von Krankheiten verwenden, insbesondere bei der Antagonisierung der zentral dämpfenden, muskelrelaxierenden, ataktischen, blutdrucksenkenden und atemdepressiven Eigenschaften von tranquilisierend wirksamen 1,4-Benzodiazepinen. Insbesondere kann man Verbindungen der allgemeinen Formel I in Kombination mit den weiter oben erwähnten schistosomizid wirksamen Verbindungen, z. B. in Kombination mit (+)-5-(o-Chlorphenyl)-1,3-dihydro-3-methyl-7-nitro-2H-1,4-benzodiazepin-2-on bei der Bekämpfung der Schistosomiasis verwenden. Dabei können die Verbindungen der Formel I oder deren pharmazeutisch annehmbare Säureadditionssalze vor, gleichzeitig mit oder nach der Verabreichung bzw. Einnahme von tranquilisierend wirksamen 1,4-Benzodiazepinen verabreicht werden. Wird

die Verbindung der Formel I oder ein pharmazeutisch annehmbares Säureadditionssalz davon gleichzeitig mit dem tranquilisierend wirksamen 1,4-Benzodiazepin verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Säureadditionssalz davon und ein tranquilisierend wirksames 1,4-Benzodiazepin-Derivat enthält; derartige pharmazeutische Kombinationen sind ebenfalls Gegenstand der vorliegenden Erfindung. Die Dosierung der Verbindungen der Formel I und ihrer pharmazeutisch annehmbaren Säureadditionssalze kann innerhalb weiter Grenzen variieren und ist natürlich in jedem einzelnen Fall den individuellen Gegebenheiten anzupassen. Im allgemeinen dürfte eine Tagesdosis von etwa 0,2 bis etwa 500 mg angemessen sein.

Wie eingangs erwähnt, sind Arzneimittel, enthaltend eine Verbindung der allgemeinen Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Im speziellen sind pharmazeutische Kombinationen, enthaltend eine Verbindung der allgemeinen Formel I und eine der weiter oben erwähnten schistosomizid wirksamen Verbindungen, insbesondere das (+)-5-(o-Chlorphenyl)-1,3-dihydro-3-methyl-7-nitro-2H-1,4-benzodiazepin-2-on, Gegenstand der vorliegenden Erfindung; derartige Kombinationen eignen sich zur Bekämpfung von Schistosomiasis.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) 11,3 g (0,057 Mol) 6-Chlorisatosäureanhydrid und 5,78 g (0,057 Mol) L-Azetidincarbonsäure werden in 50 ml Dimrethylsulfoxid während 2 Stunden auf 125° erhitzt. Anschließend dampft man im Hochvakuum zur Trockene ein und erhitzt den erhaltenen Rückstand während 2 Stunden auf 150°. Durch Chromatographieren an Kieselgel unter Eluieren mit Essigester erhält man (S)-5-Chlor-1,10a-dihydroazeto[2,1-c][1,4]benzodiazepin-4,10(2H,9H)-dion vom Schmelzpunkt 225—228°.

b) Eine Suspension von 1,30 g (29,8 mMol) Natriumhydrid (55proz. Öldispersion) in 40 ml trockenem Dimethylformamid wird bei —15° mit 6,38 g (27 mMol) (S)-5-Chlor-1,10a-dihydroazeto[2,1-c]-[1,4]benzodiazepin-4,10(2H,9H)-dion versetzt und während 0,5 Stunden bei dieser Temperatur gerührt. Anschließend kühlt man auf —35° ab und versetzt tropfenweise mit 4,8 ml (29,8 mMol) Diäthylchlorphosphat.

Inzwischen wird eine Lösung von 3,55 g (32,4 mMol) Kalium-t-butylat in 14 ml trockenem Dimethylformamid in einem Aceton/Trockeneisbad abgekühlt, mit 4,1 ml (32,4 mMol) Isocyanessigsäure-äthylester versetzt und bei —10° dem obigen Reaktionsgemisch zugetropft. Man entfernt das Kühlbad, rührt noch während ca. 15 Minuten, neutralisiert mit Eisessig, gießt auf 100 ml Wasser und extrahiert dreimal mit Chloroform. Die vereinigten Chloroformauszüge werden dreimal mit Wasser gewaschen, über Magnesiumsulfat getrocknet und eingedampft. Das erhaltene Rohprodukt wird an Kieselgel chromatographiert und anschließend aus Essigester umkristallisiert. Man erhält Äthyl-(S)-8-chlor-12,12a-dihydro-9-oxo-9H,11H-azeto[2,1-c]imidazo[1,5-a][1,4]benzodiazepin-1-carboxylat vom Schmelzpunkt 184—185°.

## Beispiel 2

a) Eine Mischung aus 1,79 g (5,4 mMol) Äthyl-(S)-8-chlor-12,12a-dihydro-9-oxo-9H,11H-azeto-[2,1-c]imidazo[1,5-a][1,4]benzodiazepin-1-carboxylat und 0,324 g (8,1 mMol) Natriumhydroxid wird mit 8,5 ml Wasser und 27 ml Äthanol versetzt und während 30 Minuten unter Rückfluß zum Sieden erhitzt. Anschließend neutralisiert man mit 8,1 ml 1 N Salzsäure, destilliert das Äthanol ab, verdünnt mit ca. 70 ml Wasser und läßt während 1 Stunde im Eisbad stehen. Das ausgefallene Material wird abgenutscht, mit Wasser gewaschen und getrocknet. Man erhält (S)-8-Chlor-12,12a-dihydro-9-oxo-9H,11H-azeto[2,1-c]imidazo[1,5-a][1,4]benzodiazepin-1-carbonsäure vom Schmelzpunkt 244—245°.

b) 0,92 g (3,0 mMol) (S)-8-Chlor-12,12a-dihydro-9-oxo-9H,11H-azeto[2,1-c]imidazo[1,5-a][1,4]-benzodiazepin-1-carbonsäure werden mit dem Bunsenbrenner bis zur Beendigung der Gasentwicklung erhitzt. Das Rohprodukt wird unter Eluieren mit Chloroform/Methanol (9:1) an Kieselgel chromatographiert. Nach Umkristallisieren aus Essigester/Hexan erhält man (S)-8-Chlor-12,12a-dihydro-9H,11H-azeto[2,1-c]imidazo[1,5-a][1,4]benzodiazepin-9-on vom Schmelzpunkt 207—208°.

## Beispiel 3

a) 16,3 g (0,1 Mol) Isatosäureanhydrid und 10,1 g (0,1 Mol) L-Azetidincarbonsäure werden in 50 ml Dimethylsulfoxid während 2 Stunden auf 115° erhitzt. Anschließend gießt man auf 450 ml Wasser, kühlt auf 0° ab und filtriert das ausgefallene kristalline Material ab. Man erhält (S)-1,10a-Dihydroazeto[2,1-c][1,4]benzodiazepin-4,10(2H,9H)-dion vom Schmelzpunkt 217—218°.

b) Eine Suspension von 1,03 g (23,7 mMol) Natriumhydrid (55proz. Öldispersion) in 25 ml trockenem Dimethylformamid wird bei —10 bis —20° mit 4,0 g (19,8 mMol) (S)-1,10a-Dihydroazeto[2,1-c]-[1,4]benzodiazepin-4,10(2H,9H)-dion versetzt und 45 Minuten bei dieser Temperatur gerührt. Das Gemisch wird anschließend bei —35° tropfenweise mit 3,2 ml (19,8 mMol) Diäthylchlorphosphat versetzt. Man rührt noch ca. 20 Minuten bei —20°.

Inzwischen wird eine Lösung von 2,17 g (19,8 mMol) Kalium-t-butylat in 7 ml trockenem Dimethylformamid in einem Aceton/Trockeneisbad abgekühlt, mit 2,5 ml (19,8 mMol) Isocyanessigsäure-äthylester versetzt und bei —15 bis —10° dem obigen Reaktionsgemisch zugetropft. Man entfernt das Kühlbad, neutralisiert nach 15 Minuten mit Eisessig, gießt auf 100 ml Wasser und extrahiert dreimal mit Chloroform. Die Chloroformauszüge werden dreimal mit Wasser gewaschen, über Magnesiumsulfat getrocknet und eingedampft. Das erhaltene Rohprodukt wird an einer Kieselgelsäule chromatographiert. Nach Umkristallisieren aus Essigester erhält man Äthyl-(S)-12,12a-dihydro-9-oxo-9H,11H-azeto[2,1-c]imidazo[1,5-a][1,4]benzodiazepin-1-carboxylat vom Schmelzpunkt 191—194°.

## Beispiel 4

a) Eine Suspension von 1,93 g (44,3 mMol) Natriumhydrid (55proz. Öldispersion) in 45 ml trockenem Dimethylformamid wird bei —10 bis —20° mit 7,78 g (38,5 mMol) (S)-1,10a-Dihydroazeto[2,1-c]-[1,4]benzodiazepin-4,10(2H,9H)-dion versetzt. Man rührt während 1,25 Stunden bei —20 bis —10°, versetzt bei —35° tropfenweise mit 7,2 ml (44,3 mMol) Diäthylchlorphosphat und rührt noch während ca. 20 Minuten bei dieser Temperatur.

Inzwischen wird eine Lösung von 4,85 g (44,3 mMol) Kalium-t-butylat in 15 ml trockenem Dimethylformamid in einem Aceton/Trockeneisbad abgekühlt, mit 6,26 g (44,3 mMol) Isocyanessigsäure-t-butylester versetzt und bei —15° dem obigen Reaktionsgemisch zugetropft. Man entfernt das Kühlbad, rührt noch während 15 Minuten, neutralisiert mit Eisessig, gießt auf 350 ml Wasser und extrahiert mehrmals mit Chloroform. Die Chloroformauszüge werden dreimal mit Wasser gewaschen, über Magnesiumsulfat getrocknet und eingedampft. Durch Chromatographieren an Kieselgel unter Eluieren mit Essigester/Chloroform (1 : 3) und anschließendes Umkristallisieren des erhaltenen Materials aus Essigester/n-Hexan erhält man t-Butyl-(S)-12,12a-dihydro-9-oxo-9H,11H-azeto[2,1-c]imidazo-[1,5-a][1,4]benzodiazepin-1-carboxylat vom Schmelzpunkt 185—187°.

b) 1,77 g (5,4 mMol) t-Butyl-(S)-12,12a-dihydro-9-oxo-9H,11H-azeto[2,1-c]imidazo[1,5-a][1,4]-benzodiazepin-1-carboxylat werden in 14 ml Trifluoressigsäure während 3 Stunden bei 50° und während 3,5 Stunden bei 65° gerührt. Anschließend wird zur Trockne eingedampft und der Rückstand während 2,5 Stunden im Hochvakuum auf dem Dampfbad erhitzt. Man versetzt mit Äther, rührt während 1 Stunde unter Eiskühlung, nutscht das feste Material unter Nachwaschen mit Äther ab und trocknet es. Man erhält (S)-12,12a-Dihydro-9-oxo-9H,11H-azeto[2,1-c]imidazo[1,5-a]-[1,4]benzodiazepin-1-carbonsäure vom Schmelzpunkt 232°.

c) 1,3 g (4,8 mMol) (S)-12,12a-Dihydro-9-oxo-9H,11H-azeto[2,1-c]imidazo[1,5-a][1,4]benzodiazepin-1-carbonsäure werden bis zur Beendigung der Gasentwicklung auf 240° erhitzt. Nach Umkristallisieren aus Essigester erhält man (S)-12,12a-Dihydro-9H,11H-azeto[2,1-c]imidazo-[1,5-a][1,4]benzodiazepin-9-on vom Schmelzpunkt 195—197°.

## Beispiel 5

0,54 g (2,4 mMol) (S)-12,12a-Dihydro-9H,11H-azeto-[2,1-c]imidazo[1,5-a][1,4]benzodiazepin-9-on und 0,32 g (2,4 mMol) N-Chlorsuccinimid werden mit 10 ml Dimethylformamid versetzt und 40 Minuten bei 90° gerührt. Das Reaktionsgemisch wird auf 50 ml Wasser gegossen und viermal mit Chloroform ausgeschüttelt. Die vereinigten Chloroformauszüge werden dreimal mit Wasser gewaschen, über Magnesiumsulfat getrocknet und eingedampft. Das Rohprodukt wird an Kieselgel unter Eluieren mit Chloroform/Methanol (19 : 1) chromatographiert und anschließend aus Essigester umkristallisiert. Man erhält (S)-1-Chlor-12,12a-dihydro-9H,11H-azeto[2,1-c]imidazo[1,5-a][1,4]benzodiazepin-9-on vom Schmelzpunkt 210—214°.

Beispiel 6

a) 2,3 g (22,8 mMol) Azetidin-2-carbonsäure und 3,72 g (22,8 mMol) Isatosäureanhydrid werden mit 20 ml Dimethylsulfoxid versetzt, während 2 Stunden bei 110° gerührt, anschließend auf 250 ml Wasser gegossen und noch während 2 Stunden gerührt. Das ausgefallene Material wird abgenutscht, mit Wasser gewaschen und aus Äthanol umkristallisiert. Man erhält (R,S)-1,10a-Dihydroazeto[2,1-c][1,4]benzodiazepin-4,10(2 H,9 H)-dion von Schmelzpunkt 250—252°.

b) Zu einer Suspension von 0,82 g (18,7 mMol) Natriumhydrid (55proz. Öldispersion) in 25 ml trockenem Dimethylformamid gibt man 3,15 g (15,6 mMol) (R,S)-1,10a-Dihydroazeto[2,1-c]-[1,4]benzodiazepin-4,10(2 H,9 H)-dion. Man rührt während 30 Minuten und versetzt anschließend bei —35° tropfenweise mit 3 ml (18,7 mMol) Diäthylchlorphosphat.

Inzwischen wird eine Lösung von 2,05 g (18,7 mMol) Kalium-t-butylat in 7 ml trockenem Dimethylformamid in einem Aceton/Trockeneisbad abgekühlt, mit 2,4 ml (18,7 mMol) Isocyanessigsäure-äthylester versetzt und bei —20° bis —10° dem obigen Reaktionsgemisch zugetropft. Anschließend entfernt man das Kühlbad, neutralisiert bei Raumtemperatur mit Eisessig, gießt auf 200 ml Wasser und extrahiert viermal mit Chloroform. Die Chloroformauszüge werden dreimal mit Wasser gewaschen, über Magnesiumsulfat getrocknet und eingedampft. Durch Chromatographieren an einer Kieselgelsäule unter Eluieren mit Essigester und anschließendes Umkristallisieren aus Essigester erhält man Äthyl-(R,S)-12,12a-dihydro-9-oxo-9 H,11 H-azeto[2,1-c]imidazo[1,5-a][1,4]benzodiazepin-1-carboxylat vom Schmelzpunkt 153—154°.

Äthyl-(S)-8-Chlor-12,12a-dihydro-9-oxo-9 H,11 H-azeto[2,1-c]imidazo[1,5-a][1,4]benzodiazepin-1-carboxylat (Wirkstoff A) kann wie in den Beispielen A bis G angegeben als Wirkstoff zur Herstellung von pharmazeutischen Präparaten verwendet werden:

Beispiel A

Es werden in üblicher Weise Tabletten folgender Zusammensetzung hergestellt:

| | | mg/Tablette |
|---|---|---|
| Wirkstoff A | | 1 |
| Milchzucker | | 103 |
| Maisstärke | | 25 |
| Mikrokristalline Cellulose | | 70 |
| Magnesiumstearat | | 1 |
| | Total | 200 |

Beispiel B

Es werden Kapseln folgender Zusammensetzung hergestellt:

| | | mg/Kapsel |
|---|---|---|
| Wirkstoff A | | 1 |
| Milchzucker | | 164 |
| Maisstärke | | 30 |
| Talk | | 5 |
| | Total | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

Beispiel C

Es werden Injektionslösungen folgender Zusammensetzung hergestellt:

| | pro ml |
|---|---|
| Wirkstoff A | 0,5 mg |
| Benzylalkohol | 0,015 ml |

| | pro ml |
|---|---|
| Propylenglykol | 0,4 ml |
| Äthanol (95proz.) | 0,1 ml |
| Natriumbenzoat | 48,8 mg |
| Benzoesäure | 1,2 mg |
| Wasser für Injektion p.s. ad | 1,0 ml |

Zur Herstellung von 10 000 ml Injektionslösung löst man 5 g des Wirkstoffes in 150 ml Benzylalkohol und gibt 4000 ml Propylenglykol und 1000 ml Äthanol zu. Dann löst man 12 g Benzoesäure in dem obigen Gemisch und gibt eine Lösung von 488 g Natriumbenzoat in 300 ml Wasser (für Injektion) zu. Die erhaltene Lösung wird durch Zugabe von Wasser (für Injektion) auf ein Volumen von 10 000 ml gebracht, filtriert und in Ampullen geeigneter Größe abgefüllt; man füllt das Restvolumen der Ampullen mit Stickstoff, schmilzt die Ampullen zu und sterilisiert sie während 30 Minuten im Autoklaven bei 0,7 atm.

Beispiel D

Es werden Suppositorien folgender Zusammensetzung hergestellt:

| | g/Supp. |
|---|---|
| Wirkstoff A | 0,001 |
| Cacaobutter (Smp. 36–37°) | 1,255 |
| Carnauba-Wachs | 0,044 |
| Total | 1,3 |

Cacaobutter und Carnauba-Wachs werden in einem Glas- oder Stahlgefäß geschmolzen, gründlich vermengt und auf 45° abgekühlt. Hierauf gibt man den fein pulverisierten Wirkstoff zu und rührt, bis es vollständig dispergiert ist. Man gießt die Mischung in Suppositorienformen geeigneter Größe, läßt abkühlen, nimmt dann die Suppositorien aus den Formen und verpackt sie einzeln in Wachspapier oder Metallfolien.

Beispiel E

Es werden Kapseln folgender Zusammensetzung hergestellt:

| | mg/Kapsel |
|---|---|
| Wirkstoff A | 20,0 |
| (+)-5-(o-Chlorphenyl)-1,3-dihydro-3-methyl-7-nitro-2 H-1,4-benzodiazepin-2-on (Wirkstoff B) | 30,0 |
| Milchzucker krist. | 100,0 |
| Maisstärke weiß | 27,5 |
| Talk | 10,0 |
| Magnesiumstearat | 2,5 |
| Total | 190,0 |

Die beiden Wirkstoffe werden mit den Hilfsstoffen gut vermischt und zu je 190,0 mg in Steckkapseln geeigneter Größe abgefüllt.

Beispiel F

Es werden Tabletten folgender Zusammensetzung hergestellt:

| | mg/Tablette |
|---|---|
| Wirkstoff A | 10,0 |
| (+)-5-(o-Chlorphenyl)-1,3-dihydro-3-methyl-7-nitro-2 H-1,4-benzodiazepin-2-on (Wirkstoff B) | 30,0 |
| Milchzucker pulvis | 15,0 |
| Maisstärke weiß | 19,5 |

| | mg/Tablette |
|---|---|
| Povidon K 30 | 3,5 |
| Maisstärke weiß | 10,0 |
| Magnesiumstearat | 2,0 |
| Total | 90,0 |

Die beiden Wirkstoffe, Milchzucker pulvis und die erste Portion Maisstärke weiß werden gemischt und gesiebt. Diese Mischung wird mit einer Lösung von Povidon K 30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 90 mg schweren Tabletten verpreßt.

Beispiel G

Es werden Tabletten folgender Zusammensetzung hergestellt:

| | mg/Tablette |
|---|---|
| Wirkstoff A | 30 |
| (+)-5-(o-Chlorphenyl)-1,3-dihydro-3-methyl-7-nitro-2 H-1,4-benzodiazepin-2-on (Wirkstoff B) | 30 |
| Milchzucker pulvis | 22 |
| Maisstärke weiß | 22 |
| Povidon K 30 | 6 |
| Maisstärke weiß | 16 |
| Magnesiumstearat | 4 |
| Total | 130 |

Die beiden Wirkstoffe, Milchzucker pulvis und die erste Portion Maisstärke weiß werden gemischt und gesiebt. Diese Mischung wird mit einer Lösung von Povidon K 30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 130 mg schweren Tabletten verpreßt.

**Patentansprüche**

1. Imidazodiazepine der allgemeinen Formel

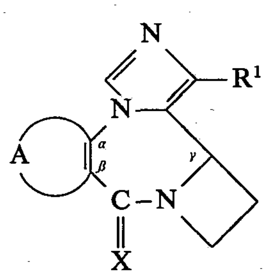

(I)

worin

A zusammen mit den beiden mit $\alpha$ und $\beta$ bezeichneten Kohlenstoffatomen die Gruppe

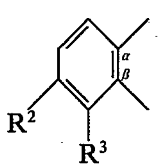

(a) oder

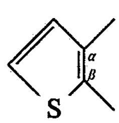

(b)

$R^1$ Wasserstoff, niederes Alkyl, niederes Alkoxymethyl, Halogen, Nitro oder einen Rest der Formel $-COOR^4$,

$R^2$ Wasserstoff, Trifluormethyl oder Halogen,
$R^3$ Wasserstoff, Trifluormethyl, Halogen oder niederes Alkyl,
$R^4$ Methyl, Äthyl oder Isopropyl und
X ein Sauerstoff- oder Schwefelatom bedeuten und das mit
$\gamma$ bezeichnete Kohlenstoffatom die (S)- oder (R,S)-Konfiguration aufweist und die mit nieder bezeichneten Reste höchstens 7 Kohlenstoffatome besitzen,

und pharmazeutisch annehmbare Säureadditionssalze davon.

2. Verbindungen gemäß Anspruch 1, dadurch gekennzeichnet, daß $R^1$ Wasserstoff, Chlor oder einen Rest der Formel $-COOR^4$ und $R^4$ Äthyl bedeuten.

3. Verbindungen gemäß Anspruch 1 oder 2, dadurch gekennzeichnet, daß A die in Anspruch 1 dargestellte Gruppe (a) bedeutet, wobei $R^2$ Wasserstoff und $R^3$ Wasserstoff oder Chlor bedeuten.

4. Verbindungen gemäß einem der Ansprüche 1 bis 3, dadurch gekennzeichnet, daß X ein Sauerstoffatom bedeutet.

5. Äthyl-(S)-8-chlor-12,12a-dihydro-9-oxo-9H,11H-azeto[2,1-c]imidazo[1,5-a][1,4]benzodiazepin-1-carboxylat,

6. Äthyl-(R,S)-12,12a-dihydro-9-oxo-9H,11H-azeto[2,1-c]imidazo[1,5-a][1,4]benzodiazepin-1-carboxylat.
(S)-8-Chlor-12,12a-dihydro-9H,11H-azeto[2,1-c]imidazo[1,5-a][1,4]benzodiazepin-9-on,
Äthyl-(S)-12,12a-dihydro-9-oxo-9H,11H-azeto[2,1-c]imidazo[1,5-a][1,4]benzodiazepin-1-carboxylat und
(S)-1-Chlor-12,12a-dihydro-9H,11H-azeto[2,1-c]imidazo[1,5-a][1,4]benzodiazepin-9-on.

7. Verbindungen der allgemeinen Formel

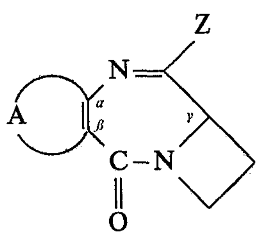

(II)

worin

A die in Anspruch 1 angegebenen Bedeutung besitzt und
Z eine Abgangsgruppe bedeutet.

8. Verbindungen der allgemeinen Formel

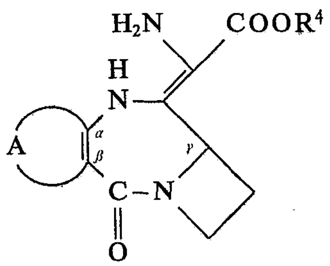

(IV)

worin

A die in Anspruch 1 angegebene Bedeutung besitzt und
$R^4$ Methyl, Äthyl oder Isopropyl bedeutet.

9. Verbindungen der allgemeinen Formel

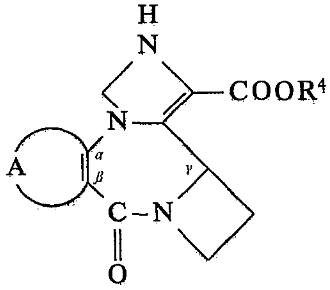

(V) und

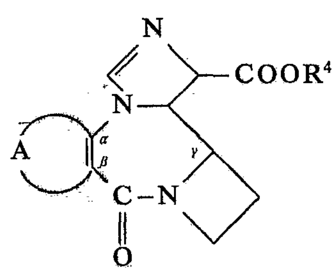

(VI)

worin

A die in Anspruch 1 angegebene Bedeutung besitzt und
$R^4$ Methyl, Äthyl oder Isopropyl bedeutet.

10. Verbindungen der allgemeinen Formel

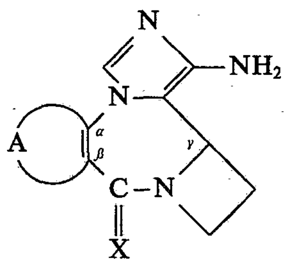

(VII)

worin

A die in Anspruch 1 angegebene Bedeutung besitzt und
X ein Sauerstoff- oder Schwefelatom bedeutet.

11. Verbindungen der allgemeinen Formel

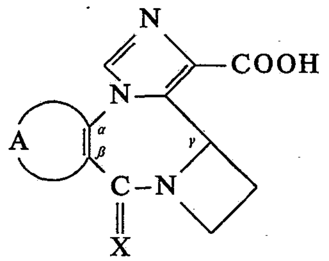

(VIII)

worin

A die in Anspruch 1 angegebene Bedeutung besitzt und
X ein Sauerstoff- oder Schwefelatom bedeutet.

12. Verbindungen der allgemeinen Formel

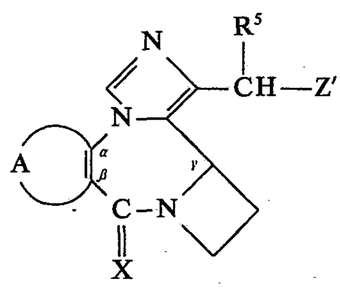

(IX)

worin

A die in Anspruch 1 angegebene Bedeutung besitzt,
X ein Sauerstoff- oder Schwefelatom,
$R^5$ Wasserstoff oder niederes Alkyl und
Z′ eine Abgangsgruppe bedeuten.

13. Verbindungen der allgemeinen Formel

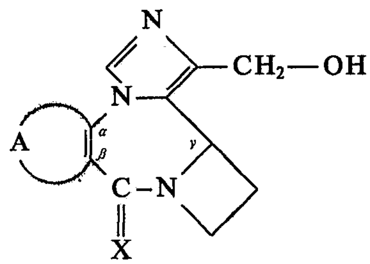

(XVI)

worin

A die in Anspruch 1 angegebene Bedeutung besitzt und
X ein Sauerstoff- oder Schwefelatom bedeutet.

14. Verbindungen der allgemeinen Formel

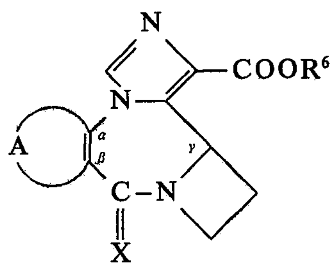

(Ib)

worin

A die in Anspruch 1 angegebene Bedeutung besitzt,
X ein Sauerstoff- oder Schwefelatom und
$R^6$ niederes Alkyl bedeuten, mit der Maßgabe, daß $R^6$ nicht Methyl, Äthyl, Isopropyl oder t-Butyl bedeutet.

15. Verbindungen gemäß einem der Ansprüche 1 bis 6 als pharmazeutische Wirkstoffe.

16. Pharmazeutische Wirkstoffe gemäß Anspruch 15, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

17. Verfahren zur Herstellung von Verbindungen gemäß einem der Ansprüche 1 bis 6, dadurch gekennzeichnet, daß man

a) eine Verbindung der allgemeinen Formel

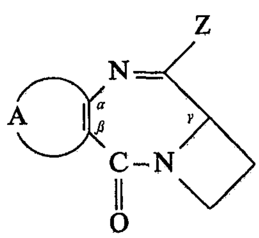

(II)

worin

A die in Anspruch 1 angegebene Bedeutung besitzt und
Z eine Abgangsgruppe bedeutet,

in Gegenwart einer Base mit einem Isocyanessigester der allgemeinen Formel

$$CN—CH_2—COOR^4 \quad (III)$$

worin $R^4$ die in Anspruch 1 angegebene Bedeutung besitzt,

umsetzt, oder

b) eine Verbindung der allgemeinen Formel

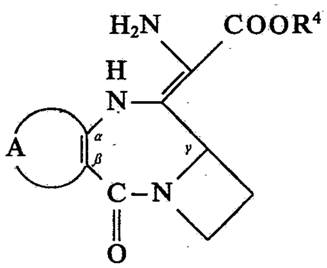

(IV)

worin A und $R^4$ die in Anspruch 1 angegebene Bedeutung besitzen, mit einem Formylierungsmittel behandelt, oder

c) eine Verbindung der allgemeinen Formel

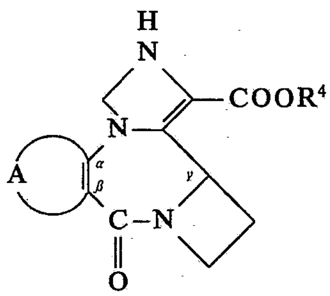

(V) oder

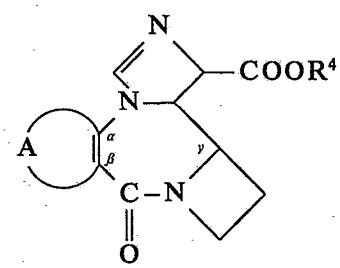

(VI)

worin A und $R^4$ die in Anspruch 1 angegebene Bedeutung besitzen, dehydriert, oder

d) in einer Aminoverbindung der allgemeinen Formel

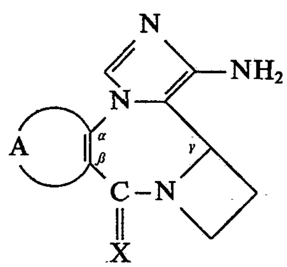

(VII)

worin A und X die in Anspruch 1 angegebene Bedeutung besitzen, die Aminogruppe durch ein Halogenatom oder durch die Nitrogruppe ersetzt, oder

e) in einer Aminoverbindung der obigen allgemeinen Formel VII die Aminogruppe zur Nitrogruppe oxidiert, oder

f) eine Carbonsäure der allgemeinen Formel

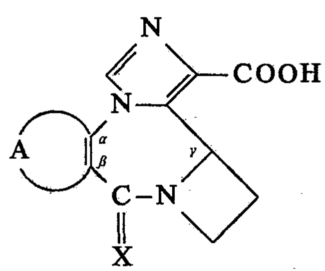

(VIII)

worin A und X die in Anspruch 1 angegebene Bedeutung besitzen,

decarboxyliert, oder

g) eine Verbindung der allgemeinen Formel

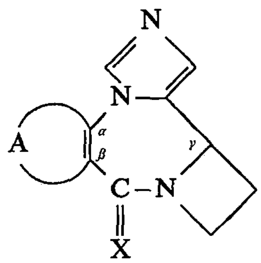

(Ia)

worin A und X die in Anspruch 1 angegebene Bedeutung besitzen,

am Imidazolring halogeniert, oder

h) in einer Verbindung der allgemeinen Formel

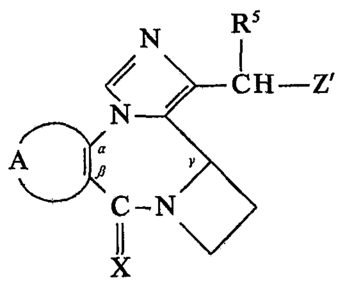

(IX)

worin

A und X die in Anspruch 1 angegebene Bedeutung besitzen,
$R^5$ Wasserstoff oder niederes Alkyl und
Z' eine Abgangsgruppe bedeuten,

die mit Z' bezeichnete Abgangsgruppe unter reduktiven Bedingungen abspaltet, oder

i) eine Verbindung der allgemeinen Formel

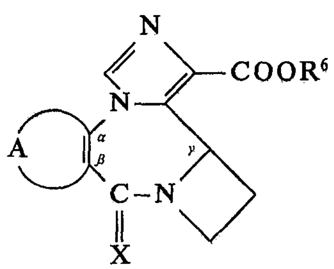

(Ib)

worin

A und X die in Anspruch 1 angegebene Bedeutung besitzen und
$R^6$ niederes Alkyl bedeutet,

umestert, oder

j) in einer Verbindung der allgemeinen Formel

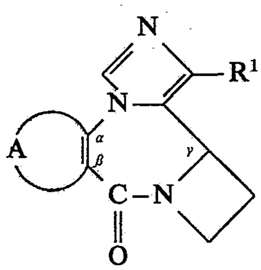

(Ic)

worin $R^1$ und A die in Anspruch 1 angegebene Bedeutung besitzen, die Carbonylgruppe in die Thiocarbonylgruppe überführt, oder

k) eine Verbindung der allgemeinen Formel

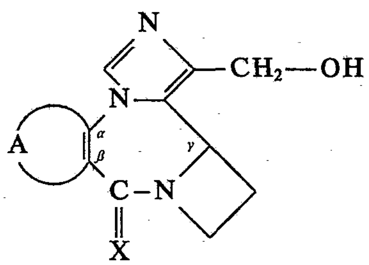

(XVI) bzw.

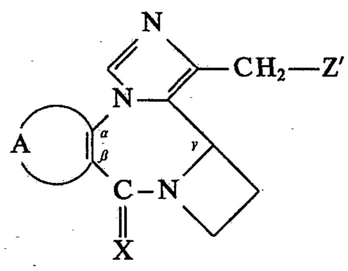

(IXa)

worin

A und X die in Anspruch 1 angegebene und
Z′ obige Bedeutung besitzen,

mit einem einen niederen Alkylrest liefernden Alkylierungsmittel, bzw. mit einem niederen Alkohol veräthert, und

l) erwünschtenfalls eine erhaltene Verbindung der allgemeinen Formel I in ein pharmazeutisch annehmbares Säureadditionssalz überführt.

18. Arzneimittel, enthaltend eine Verbindung gemäß einem der Ansprüche 1 bis 6.

19. Arzneimittel gemäß Anspruch 18, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

20. Schistosomizid wirksame Arzneimittel, enthaltend eine Verbindung gemäß einem der Ansprüche 1 bis 6 und (+)-5-(o-Chlorphenyl)-1,3-dihydro-3-methyl-7-nitro-2H-1,4-benzodiazepin-2-on.

## Claims

1. Imidazodiazepines of the general formula

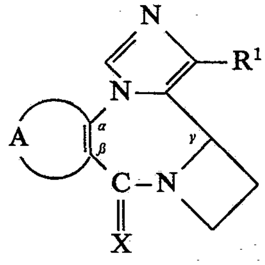

(I)

wherein

A together with the two carbon atoms denoted as $\alpha$ and $\beta$ signifies the group

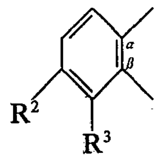

(a) or (b)

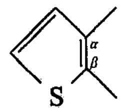

$R^1$ signifies hydrogen, lower alkyl, lower alkoxymethyl, halogen, nitro or a residue of the formula $-COOR^4$.
$R^2$ signifies hydrogen, trifluoromethyl or halogen,
$R^3$ signifies hydrogen, trifluoromethyl, halogen or lower alkyl,
$R^4$ signifies methyl, ethyl or isopropyl and
X signifies an oxygen or sulphur atom, and the carbon atom denoted as
$\gamma$ has the (S)- or (R,S)-configuration and the residues denoted as lower have a maximum of 7 carbon atoms,

and pharmaceutically acceptable acid addition salts thereof.

2. Compounds in accordance with claim 1, characterized in that $R^1$ signifies hydrogen, chlorine or a residue of the formula $-COOR^4$ and $R^4$ signifies ethyl.

3. Compounds in accordance with claim 1 or 2, characterized in that A signifies the group (a) depicted in claim 1 in which $R^2$ signifies hydrogen and $R^3$ signifies hydrogen or chlorine.

4. Compounds in accordance with any one of claims 1 to 3, characterized in that X signifies an oxygen atom.

5. Ethyl (S)-8-chloro-12,12a-dihydro-9-oxo-9H,11H-azeto[2,1-c]imidazo[1,5-a][1,4]benzodiazepine-1-carboxylate.

6. Ethyl (R,S)-12,12a-dihydro-9-oxo-9H,11H-azeto[2,1-c]imidazo[1,5-a][1,4]benzodiazepine-1-carboxylate,
(S)-8-chloro-12,12a-dihydro-9H,11H,azeto[2,1-c]imidazo[1,5-a][1,4]benzodiazepine-9-one,
ethyl (S)-12,12a-dihydro-9-oxo-9H,11H-azeto[2,1-c]imidazo[1,5-a][1,4]benzodiazepine-1-carboxylate and
(S)-1-chloro-12,12a-dihydro-9H,11H-azeto[2,1-c]imidazo[1,5-a][1,4]benzodiazepin-9-one.

7. Compounds of the general formula

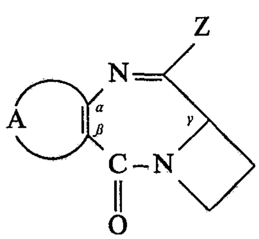

(II)

wherein

A has the significance given in claim 1 and
Z signifies a leaving group.

8. Compounds of the general formula

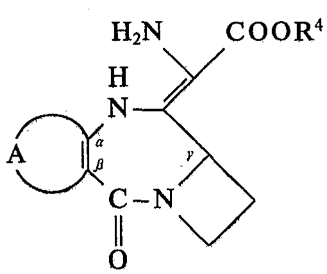

(IV)

wherein

A has the significance given in claim 1 and
$R^4$ signifies methyl, ethyl or isopropyl.

9. Compounds of the general formulae

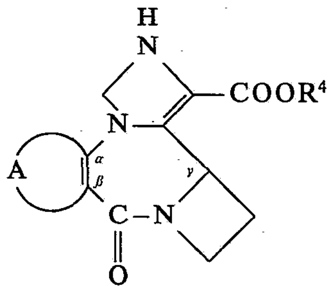

(V) and

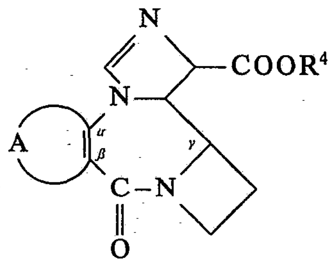

(VI)

wherein

A has the significance given in claim 1 and
$R^4$ signifies methyl, ethyl or isopropyl.

10. Compounds of the general formula

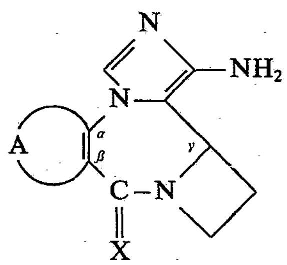

(VII)

wherein

A has the significance given in claim 1 and
X signifies an oxygen or sulphur atom.

11. Compounds of the general formula

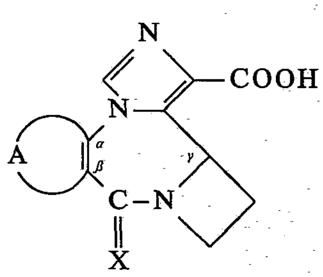

(VIII)

wherein

A has the significance given in claim 1 and
X signifies an oxygen or sulphur atom.

12. Compounds of the general formula

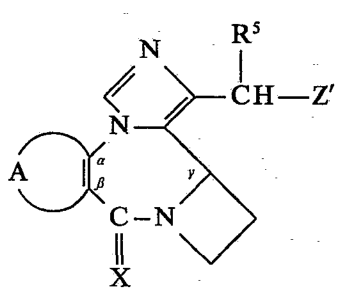

(IX)

wherein

A has the significance given in claim 1,
X signifies an oxygen or sulphur atom,
$R^5$ signifies hydrogen or lower alkyl and
Z' signifies a leaving group.

13. Compounds of the general formula

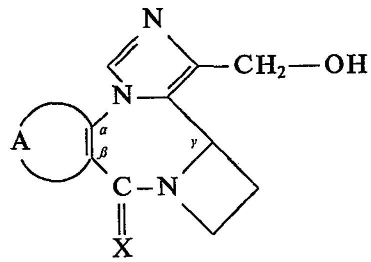

(XVI)

wherein

A has the significance given in claim 1 and
X signifies an oxygen or sulphur atom.

14. Compounds of the general formula

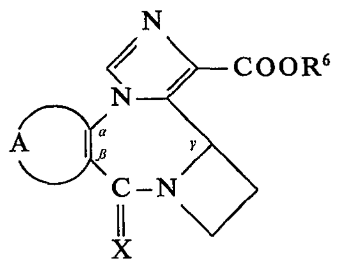

(Ib)

wherein

A has the significance given in claim 1,
X signifies an oxygen or sulphur atom and
$R^6$ signifies lower alkyl, with the proviso that $R^6$ does not signify methyl, ethyl, isopropyl or t-butyl.

15. Compounds in accordance with any one of claim 1 to 6 as pharmaceutically active substances.

16. Pharmaceutically active substances in accordance with claim 15, which antagonize the central-depressant, muscle relaxant, ataxic, blood pressure-lowering and respiratory-depressant properties of 1,4-benzodiazepines which have tranquillizing activity.

17. A process for the manufacture of compounds in accordance with any one of claims 1 to 6, characterized by

a) reacting a compound of the general formula

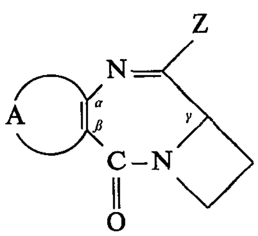

(II)

wherein

A has the significance given in claim 1 and
Z signifies a leaving group,

in the presence of base with an isocyanoacetic ester of the general formula

$CN—CH_2—COOR^4$ (III)

wherein $R^4$ has the significance given in claim 1,

or

b) treating a compound of the general formula

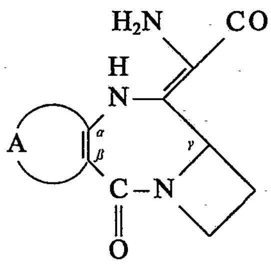

(IV)

wherein A and $R^4$ have the significance given in claim 1,

with a formylating agent, or

c) dehydrogenating a compound of the general formula

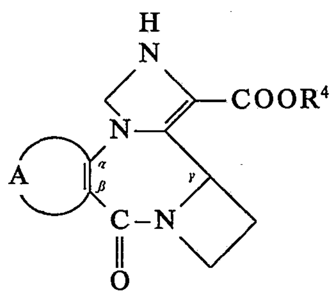

(V) or

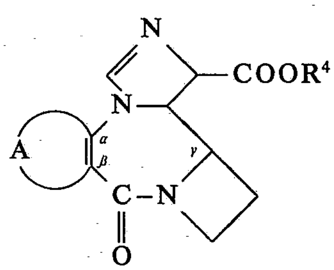

(VI)

wherein A and $R^4$ have the significance given in claim 1,

or

d) replacing the amino group in a compound of the general formula

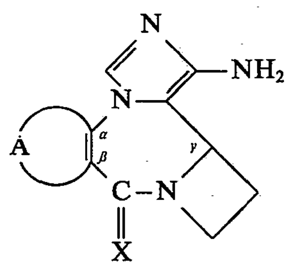

(VII)

wherein A and X have the significance given in claim 1,

by a halogen atom or by the nitro group, or

e) oxidizing the amino group in an amino compound of general formula VII above to the nitro group, or

f) decarboxylating a carboxylic acid of the general formula

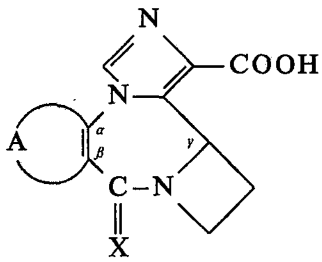

(VIII)

wherein A and X have the significance given in claim 1,

or

g) halogenating a compound of the general formula

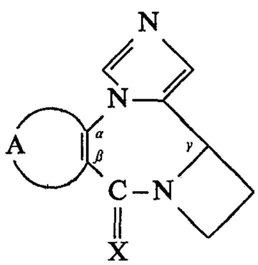

(Ia)

wherein A and X have the significance given in claim 1,

on the imidazole ring, or

h) cleaving off under reductive conditions the leaving group denoted by Z′ in a compound of the general formula

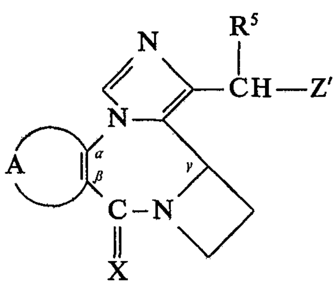

(IX)

wherein

A and X have the significance given in claim 1,
$R^5$ signifies hydrogen or lower alkyl and
Z′ signifies a leaving group,

or

i) trans-esterifying a compound of the general formula

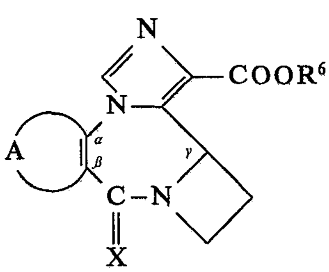

(Ib)

wherein

A and X have the significance given in claim 1 and
$R^6$ signifies lower alkyl,

or

j) converting the carbonyl group in a compound of the general formula

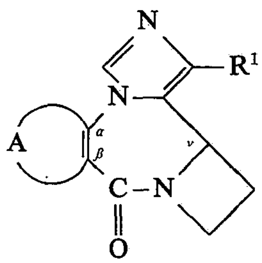

(Ic)

wherein $R^1$ and A have the significance given in claim 1,

into the thiocarbonyl group, or

k) etherifying a compound of the general formula

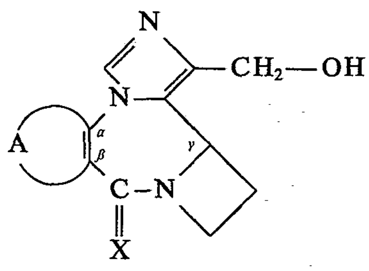

(XVI) or

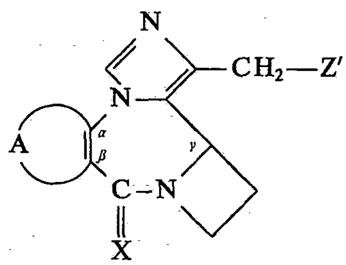

(IXa)

wherein

A and X have the significance given in claim 1 and
Z′ has the above significance,

with an alkylating agent yielding a lower alkyl residue and with a lower alcohol, respectively, and

l) if desired, converting a compound of general formula I obtained into a pharmaceutically acceptable acid addition salt.

18. Medicaments containing a compound in accordance with any one of claims 1 to 6.

19. Medicaments in accordance with claim 18, which antagonize the central-depressant, muscle relaxant, ataxic, blood pressure-lowering and respiratory-depressant properties of 1,4-benzodiazepines which have tranquillizing activity.

20. Schistosomicidally active medicaments containing a compound in accordance with any one of claims 1 to 6 and (+)-5-(o-chlorophenyl)-1,3-dihydro-3-methyl-7-nitro-2H-1,4-benzodiazepin-2-one.

**Revendications**

1. Imidazodiazépines de formule générale:

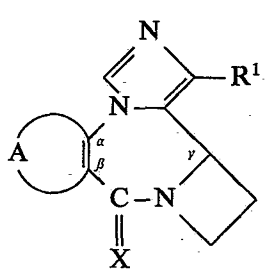

(I)

dans laquelle

A forme avec les deux atomes de carbone $\alpha$ et $\beta$ le groupe

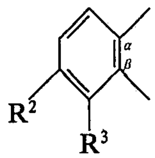

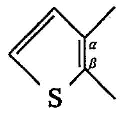

(a) ou (b)

$R^1$ représente l'hydrogène, un groupe alkyle inférieur, alcoxyméthyle inférieur, un halogène, un groupe nitro ou un reste de formule $-COOR^4$,
$R^2$ représente l'hydrogène, un groupe trifluorméthyle ou un halogène,
$R^3$ représente l'hydrogène, un groupe trifluorométhyle, un halogène ou un groupe alkyle inférieur,
$R^4$ représente un groupe méthyle, éthyle ou isopropyle et
X représente un atome d'oxygène ou de soufre, et l'atome de carbone
$\gamma$ est en configuration (S) ou (R,S), l'expression »inférieur« qualifiant des groupes qui ont 7 atomes de carbone au maximum,

et leurs sels formés par addition avec des acides acceptables pour l'usage pharmaceutique.

2. Composés selon la revendication 1, caractérisés en ce que $R^1$ représente l'hydrogène, le chlore ou un reste de formule $-COOR^4$, $R^4$ étant un groupe éthyle.

3. Composés selon la revendication 1 ou 2, caractérisés en ce que A représente le groupe (a) de la revendication 1, dans lequel $R^2$ représente l'hydrogène et $R^3$ l'hydrogène ou le chlore.

4. Composés selon l'une des revendications 1 à 3, caractérisés en ce que X représente un atome d'oxygène.

5. Le (S)-8-chloro-12,12a-dihydro-9-oxo-9H,11H-azéto[2,1-c]imidazo[1,5-a][1,4]benzodiazépine-1-carboxylate d'éthyle.

6. Le (R,S)-12,12a-dihydro-9-oxo-9H,11H-azéto[2,1-c]imidazo[1,5-a][1,4]benzodiazépine-1-carboxylate d'éthyle,
la (S)-8-chloro-12,12a-dihydro-9H,11H-azéto[2,1-c]imidazo[1,5-a][1,4]benzodiazépine-9-one,
le (S)-12,12a-dihydro-9-oxo-9H,11H-azéto[2,1-c]imidazo[1,5-a][1,4]benzodiazépine-1-carboxylate d'éthyle, et
la (S)-1-chloro-12,12a-dihydro-9H,11H-azéto[2,1-c]imidazo[1,5-a][1,4]benzodiazépine-9-one.

7. Composés de formule générale:

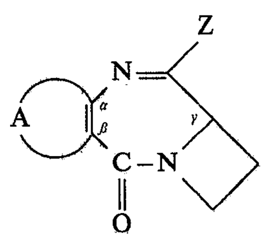

(II)

dans laquelle

A a la signification indiquée dans la revendication 1 et
Z représente un groupe éliminable.

8. Composés de formule générale:

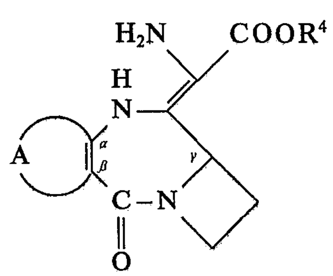

(IV)

dans laquelle

A a la signification indiquée dans la revendication 1 et
$R^4$ représente un groupe méthyle, éthyle ou isopropyle.

9. Composés de formule générale:

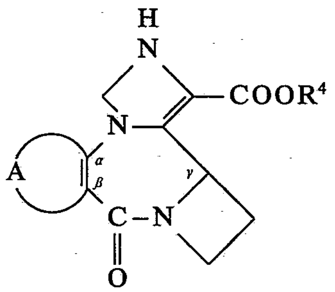

(V)

et

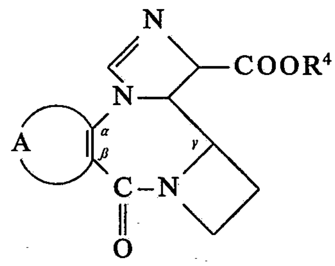

(VI)

dans laquelle

A a la signification indiquée dans la revendication 1 et
$R^4$ représente un groupe méthyle, éthyle ou isopropyle.

10. Composés de formule générale:

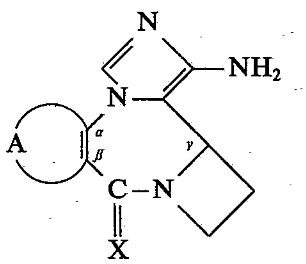

(VII)

dans laquelle

A a la signification indiquée dans la revendication 1 et
X représente un atome d'oxygène ou de soufre.

11. Composés de formule générale:

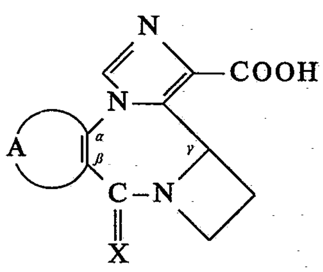

(VIII)

dans laquelle

A a la signification indiquée dans la revendication 1 et
X représente un atome d'oxygène ou de soufre.

12. Composés de formule générale:

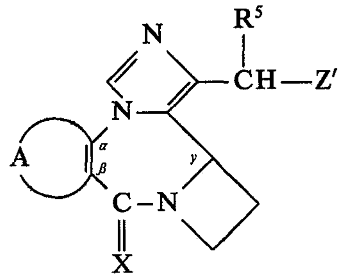

(IX)

dans laquelle

A a la signification indiquée dans la revendication 1,
X représente un atome d'oxygène ou de soufre,
$R^5$ représente l'hydrogène ou un groupe alkyle inférieur et
Z' un groupe éliminable.

13. Composés de formule générale:

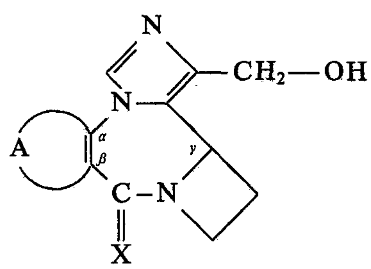

(XVI)

dans laquelle

A a la signification indiquée dans la revendication 1 et
X représente un atome d'oxygène ou de soufre.

14. Composés de formule générale:

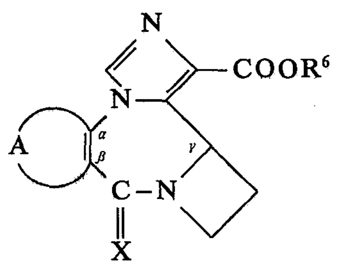

(Ib)

dans laquelle

A a la signification indiquée dans la revendication 1,
X représente un atome d'oxygène ou de soufre et
$R^6$ représente un groupe alkyle inférieur, étant spécifié que $R^6$ ne peut représenter un groupe méthyle, éthyle, isopropyle ou tert.-butyle.

15. Composés selon l'une des revendications 1 à 6, en tant que substances actives pharmaceutiques.

16. Substances actives pharmaceutiques selon la revendication 15, qui antagonisent les propriétés sédatives centrales, myorelaxantes, atactiques, hypotensives et dépressives respiratoires des 1,4-benzodiazépines à activité tranquillisante.

17. Procédé de préparation des composés selon l'une des revendications 1 à 6, caractérisé en ce que:

(a) on fait réagir un composé de formule générale:

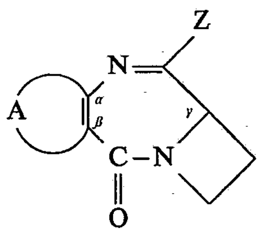

(II)

dans laquelle

A a la signification indiquée dans la revendication 1 et
Z représente un groupe éliminable,

en présence d'une base, avec un ester isocyanacétique de formule générale:

$CN—CH_2—COOR^4$ (III)

dans laquelle $R^4$ a la signification indiquée dans la revendication 1,

ou bien

(b) on traite un composé de formule générale:

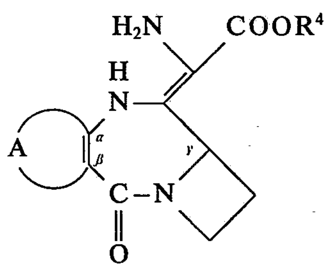

(IV)

dans laquelle A et $R^4$ ont les significations indiquées dans la revendication 1,

par un agent formylant, ou bien

(c) on soumet un composé de formule générale

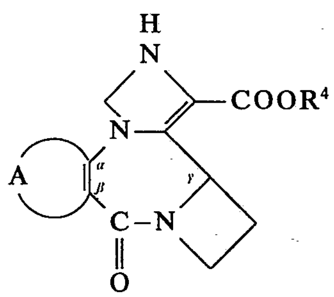

(V) ou

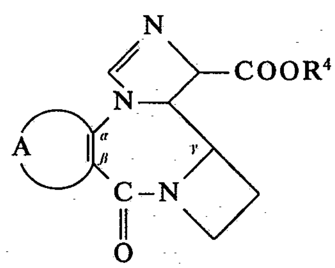

(VI)

dans laquelle A et $R^4$ ont les significations indiquées dans la revendication 1,

à déshydrogénation, ou bien

(d) dans un composé aminé de formule générale:

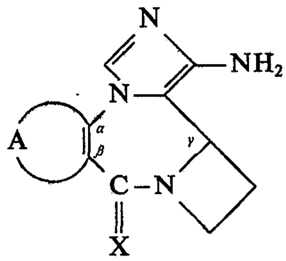

(VII)

dans laquelle A et X ont les significations indiquées dans la revendication 1,

on remplace le groupe amino par un atome d'halogène ou par le groupe nitro, ou bien

(e) dans un composé aminé de formule générale VII ci-dessus, on oxyde le groupe amino en groupe nitro, ou bien

(f) on soumet un acide carboxylique de formule générale:

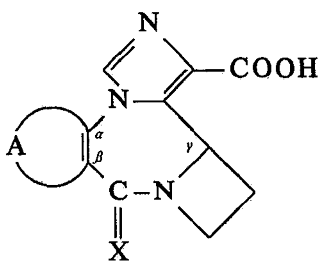

(VIII)

dans laquelle A et X ont les significations indiquées dans la revendication 1,

à décarboxylation, ou bien

(g) on halogène sur le cycle imidazole un composé de formule générale:

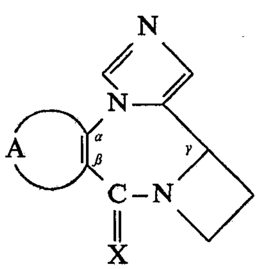

(Ia)

dans laquelle A et X ont les significations indiquées dans la revendication 1,

ou bien

(h) dans un composé de formule générale:

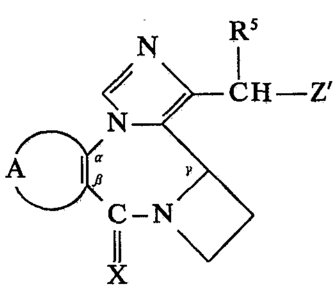

(IX)

dans laquelle

A et X ont les significations indiquées dans la revendication 1,
$R^5$ représente l'hydrogène ou un groupe alkyle inférieur et

Z' un groupe éliminable,

on élimine le groupe éliminable Z' dans des conditions réductrices, ou bien

(i) on soumet un composé de formule générale:

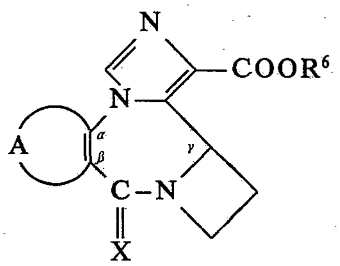

(Ib)

dans laquelle

A et X ont les significations indiquées dans la revendication 1 et
$R^6$ représente un groupe alkyle inférieur,

à transestérification, ou bien

(j) dans un composé de formule générale:

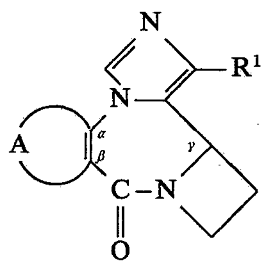

(Ic)

dans laquelle $R^1$ et A ont les significations indiquées dans la revendication 1,

on convertit le groupe carbonyle en groupe thiocarbonyle, ou bien

(k) on éthérifie un composé de formule générale:

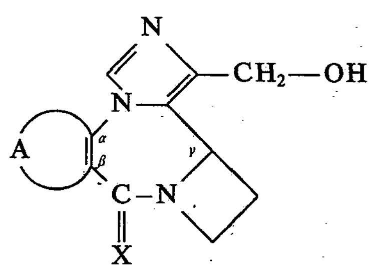

(XVI) ou

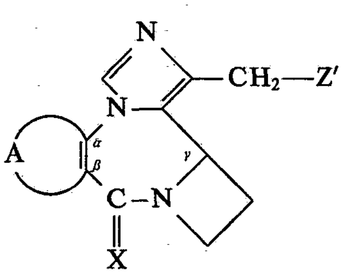

(IXa)

dans laquelle

A et X ont les significations indiquées dans la revendication 1 et
Z' a la signification indiquée ci-dessus,

par un agent alkylant fournissant un groupe alkyle inférieur ou respectivement par un alcool inférieur, et

(l) si on le désire, on convertit un composé obtenu répondant à la formule générale I en un sel par addition avec un acide acceptable pour l'usage pharmaceutique.

18. Médicaments contenant un composé selon l'une des revendications 1 à 6.

19. Médicaments selon la revendication 8, qui antagonisent les propriétés sédatives centrales, myorelaxantes, atactiques, hypotensives et dépressives respiratoires des 1,4-benzodiazépines à activité tranquillisante.

20. Médicaments à activité schistosomicide, contenant un composé selon l'une des revendications 1 à 6 et de la (+)-5-(o-chlorophényl)-1,3-dihydro-3-méthyl-7-nitro-2H-1,4-benzodiazépine-2-one.